# EUROPÄISCHE PATENTANMELDUNG

(11) **EP 2 815 787 A2**
(43) Veröffentlichungstag der Anmeldung: **24.12.2014**
(21) Anmeldenummer: 14001528.0
(22) Anmeldetag: 30.04.2014
(51) Int. Cl.: A61N 1/04, A61N 1/36

(54) **Elektrodenanordnung und Vorrichtung zur Behandlung von Schmerzen**

(30) Priorität: 30.04.2013 EP 13002304; 30.04.2013 EP 13002303
(71) Anmelder: Bomedus GmbH, 53115 Bonn (DE)
(72) Erfinder: Weigl, Johannes, 40479 Düsseldorf (DE); Weigl, Tobias, 53115 Bonn (DE); Mücke, Martin, 53545 Ockenfels (DE)
(74) Vertreter: von Renesse, Dorothea

(57) **Zusammenfassung**

Die Erfindung betrifft eine Elektrodenanordnung mit einem Elektrodenfeld sowie mindestens einer zu dem Elektrodenfeld beabstandeten, weiteren Elektrode zur Behandlung von Schmerzen eines Menschen oder eines Tieres, wobei die Elektrodenanordnung mindestens mit einem Teil zur Anlage an dem Menschen oder an das Tier ausgebildet ist, und eine Vibrationseinrichtung umfasst.

## Beschreibung

Die Erfindung betrifft eine Elektrodenanordnung und eine Vorrichtung zur Behandlung von Schmerzen.

Aus der Praxis sind verschiedene Methoden bekannt, die eine Schmerzreduktion bewirken können. Hierunter fällt neben verschiedenen durch Injektion applizierbaren Lokalanästhetika auch die häufig verwendete transdermale Lokalanästhesie. Ferner sind die lontophorese, die Verwendung von Eisspray und die Psychotherapie zur Schmerzreduktion geeignet. All diese Methoden und Verfahren sind Ressourcen aufwendig, da sie Zeit und Personal erfordern und sind dementsprechend kostentenintensiv. Ferner ist zur Schmerzreduktion die transkutane elektrische Nervenstimulation (TENS) bekannt.

US 2005/0165459 A1 beschreibt den Aufbau einer TENS Steuereinheit und einer hieran angeschlossenen Elektrode, die für die Schmerzunterdrückung bei Injektionen genutzt werden kann. Eine Elektrode kann in einer bevorzugten Ausführungsform aus konzentrischen Kreisstücken bestehen, die um eine Einstichstelle angeordnet sind. Die einzelnen kreisförmigen, nicht leitend miteinander verbundenen Elektrodenelemente können durch die TENS Steuereinheit so gesteuert werden, dass ein Reizstrom zur Schmerzunterdrückung nach Aufbringung auf die Haut um die Einstichstelle generiert werden kann. US 2005/0165459 A1 offenbart hierzu ebenfalls eine mögliche Programmierung der Steuereinheit, um die so gestalteten Elektrodenbestandteile mit einem Reizstrom zu beschalten.

WO 2008/129555 beschreibt eine Vorrichtung zur transkutanen Elektrostimulation zur Schmerzreduktion, bei der Reizströme verwendet werden, die eine effektive Schmerzunterdrückung erlauben. Die hierbei verwendeten Reizströme können eine dreieckförmige oder deltaförmige Form bei variabler Impulsdauer und Amplitude aufweisen. Die Applikation des Reizstromes erfolgt in einer bevorzugten Ausführungsform über Hautflächen von 16mm², die vorzugsweise 4cm voneinander getrennt liegen bei einem Reizstrom von 100mA. Die Reizstrompulse weisen hierbei eine variable Amplitude auf, deren Maximum ca. 100mA beträgt. In einer weiteren bevorzugten Ausführungsform liegt die Impulsbreite bei 25 - 50µs, wobei die Anstiegs- und Abfallzeit nicht mehr als 5% der Pulsbreite beträgt, mit einem Inter-Pulsinterval von 0,1 bis 3ms. Die Amplitude der Pulse wird weiterhin dahingehend moduliert, dass die jeweilige Impulsamplitude bevorzugt 70% -100% der voreingestellten Impulsamplitude beträgt.

Vor diesem Hintergrund liegt der Erfindung die Aufgabe zugrunde, eine verbesserte Vorrichtung zur Behandlung von Schmerzen, insbesondere zur Reduzierung von Schmerzen, bereitzustellen, die an einem menschlichen oder tierischen Körperteil auftreten können.

Diese Aufgabe wird durch die Gegenstände der unabhängigen Ansprüche gelöst. Vorteilhafte Ausführungsformen sind in den Unteransprüchen und der hiernach folgenden Beschreibung wiedergegeben.

Zur Behandlung, insbesondere Reduzierung oder Unterdrückung, von akuten und/oder chronischen Schmerzen schlägt die Erfindung eine Elektrodenanordnung vor. Eine Oberfläche der Elektroden ist zur Anlage auf der Haut ausgestaltet. Hierdurch kann eine einfach aufgebaute und flexibel ansteuerbare Elektrodenanordnung geschaffen werden. Durch die flexible Ansteuerbarkeit der Elektrodenanordnung ist die Möglichkeit eines weiten Anwendungsbereichs für die Schmerzbehandlung und/oder Schmerzreduzierung und/oder Schmerzunterdrückung gegeben.

Es können bevorzugt mehrere Anoden vorgesehen sein, d.h. eine Anzahl von größer gleich zwei. Ferner können mehrere Kathoden vorgesehen sein.

Im Sinne der Erfindung ist unter einer Kathode bzw. einer Anode eine Elektrode mit entsprechender Polarität zu verstehen. Vorzugsweise wechselt eine Elektrode das Vorzeichen bei einer Strom- bzw. Spannungsbeaufschlagung nicht. Es kann aber auch vorgesehen sein, dass eine Elektrode mit Strom-/Spannungspulsen beaufschlagt wird, deren Polarität sich ändert.

Im Sinne der Erfindung ist unter "Anlage auf der Haut" zu verstehen, dass die Elektroden vorzugsweise auf der Haut aufliegen und die Haut nicht perforiert bzw. auch nicht teilweise durchdrungen wird. Die Elektrodenanordnung kann an einem menschlichen oder tierischen Körper zur Auflage an der Haut getragen werden. Vorzugsweise kann die Elektrodenanordnung ohne wesentliche Einschränkung der Bewegungsfähigkeit am menschlichen oder tierischen Körper getragen werden.

Bevorzugt können die hier beschriebenen Elektrodenanordnungen in Anlage an ein Körperteil angebracht werden.

Die mehreren Anoden können als konzentrische Anodenringe um eine zentrale Kathode ausgestaltet sein. Durch diese Anordnung ist eine flexible Beaufschlagung der Anoden möglich. Es ist vorgesehen, dass mindestens ein Anodenring angesteuert wird, wobei auch optional zwei oder mehr Ringe angesteuert werden können.

Es kann auch vorgesehen sein, dass mehrere Anoden und mehrere Kathoden für eine flächige Ausgestaltung der Elektrodenanordnung vorgesehen sind. Die Anoden und Kathoden können in Form eines Elektrodenarrays angeordnet sein. Im Falle der flächigen Ausgestaltung können die Anoden und Kathoden linienförmig angeordnet sein, was zu einer einfachen Geometrie der Anordnung der Anoden und Kathoden führt und zugleich eine einfache Möglichkeit der Verbindung der Anoden bzw. Kathoden liefert. Im Sinne der Erfindung umfasst eine linienförmige Anordnung eine Anordnung in einer Reihe; gleichartig ausgestaltete Elektroden sind mit ihren Mittelpunkten wie auf einer Kette aufgereiht. Eine linienförmige Anordnung schließt auch eine wellenförmige oder zickzackförmige Anordnung ein. Die Anordnung kann auch verzerrt sein, so dass keine Zeilen oder Spalten in gerader Ausrichtung vorhanden sind, sondern eine Anordnung flächig verteilter Elektroden, die punktuell an der Haut anliegen können.

In einer weiter bevorzugten Ausführungsform können die Anoden und Kathoden quer zur linienförmigen Ausgestaltung der Anordnung quer zur Linie alternierend angeordnet sein. Hierdurch kann ein geometrisch definiertes Raster geschaffen werden mit vorbestimmbaren Eigenschaften hinsichtlich der Emission des elektrischen Feldes in die Haut. Es kann vorgesehen sein, dass bei der alternierenden Anordnung quer zur linienförmigen Ausbildung Kathoden und Anoden einander direkt gegenüber liegend, quasi gitterförmig angeordnet sein können. Es kann auch vorgesehen sein, dass linienförmig angeordnete Kathoden und Anoden versetzt zueinander vorliegen, so dass eine Kathode einem Zwischenraum zwischen zwei Anoden gegenüberliegt. Eine versetzte Anordnung scheint unbeabsichtigte Kurzschlüsse verringern und die Schmerzreduzierung und/oder Schmerzunterdrückung verbessern zu können. Es kann eine 40mm x 40mm Matrix vorgesehen sein mit 17 Reihen und 17 Spalten, wobei in den Reihen eine alternierende Abfolge von Anode und Kathode vorliegt. Die Matrixgröße bzw. die Größe der Erstreckung der Elektrodenanordnung kann je nach Anwendungsgebiet und/oder zu erfassender Hautpartie beliebig groß bzw. klein gewählt werden. Beispielsweise kann ein Flächengebilde mit einer Elektrodenanordnung eine Fläche von 10cm x 10cm deutlich überschreiten.

Es sind auch Mischformen bzw. Kombinationen aus flächenförmiger Elektrodenanordnung und Elektrodenanordnung mit konzentrischer Elektrode möglich.

In einer besonders bevorzugten Ausführungsform wird ein Elektrodenfeld als Teil der Elektrodenanordnung mit mehreren Elektroden verwendet. Die Elektroden können insbesondere als Kathoden dienen. Die Elektroden können für eine punktuelle Kontaktierung ausgestaltet sein. Eine Elektrode kann als Kontaktpunkt zur Anlage auf der Haut ausgestaltet sein. Die einzelnen Kontaktpunkte, die Elektroden, weisen jeweils einen Durchmesser von bis zu 3,5mm auf. Die einzelnen Kontaktpunkte können jeweils in einem Abstand von wenigen Millimetern bis Zentimetern, insbesondere zwischen 5,0mm bis 25mm, besonders bevorzugt 5,0mm bis 7,5mm, vorzugsweise etwa 7,5mm, angeordnet sein. Das Elektrodenfeld kann in Spalten oder in Zeilen angeordnete Elektroden aufweisen. Es kann aber auch vorgesehen sein, dass die Elektroden des Elektrodenfelds ohne erkennbar gleichmäßiges Muster flächig verteilt sind. Erfindungsgemäß wird von dem Begriff Elektrodenfeld eine flächige Verteilung von punktuell ausgebildeten Elektroden erfasst. Vorzugsweise überdeckt das Elektrodenfeld einen Bereich von 20 cm² bis 250 cm². Vorzugsweise können auf dem Feld bzw. der Fläche 2 bis 225 Elektroden ausgebildet sein. Eine Elektrodendichte des Elektrodenfelds von mindestens 1/cm², bevorzugt mindestens 2/cm², und insbesondere bevorzugt mindestens 4/cm² kann erfindungsgemäß vorgesehen sein. Es gab gute Ergebnisse bei 12 Elektroden pro 1cm².

Der Unterschied zum Elektrodenfeld und dem Elektrodenarray ist, dass die flächig verteilt angeordneten Elektroden bei dem Elektrodenfeld alle mit gleicher Polarität mit Strom bzw. Spannung beaufschlagt werden. Im Falle, dass die Elektrode des Elektrodenfelds mit Strom bzw. Spannung gleicher Polarität (beispielsweise Kathoden) beaufschlagt werden, wird mindestens eine zum Elektrodenfeld beabstandete, weitere Elektrode als Gegenelektrode (beispielsweise Anode) verwendet, die vorzugsweise für eine flächige Anlage auf der Haut ausgestaltet ist. Vorzugsweise erfolgt eine flächige Kontaktierung als Flächenelektrode oder Gelelektrode. Die Fläche der Anode als Gegenelektrode für das Elektrodenfeld als Kathode kann im Bereich des zweifachen der Fläche des Elektrodenfelds bis zum dreifachen der Fläche des Elektrodenfelds liegen. Es können auch noch größere Flächen für die Anode gewählt werden. Der Abstand zwischen Kathoden (vorzugsweise Elektrodenfeld) und Anode (Gegenelektrode) kann insbesondere im Bereich von 0,5cm bis 10cm liegen. Der Abstand zwischen Kathoden und Anode kann von der Region des Körpers abhängig gemacht werden, in der die Anlage an die Haut erfolgt. Beispielsweise kann der Abstand von 5cm bis 10cm für den Bereich des Knies und eine Behandlung von Schmerzen dort geeignet sein. Kleinere Abstände im Bereich von 0,5cm bis 4cm können beispielsweise bei der Anlage im Rückenbereich gewählt werden. Vorzugsweise ist der Abstand und die Ausrichtung und Lage der Kathoden und der Anode(n) zueinander mittels eines Haltemittels festgelegt, an dem die Kathoden und die Anode(n) angeordnet sind. Das Haltemittel kann beispielsweise eine flexible Fläche, eine Bandage, ein Band oder ein sonstiges Textil oder Gewirke sein.

Eine erfindungsgemäße Elektrodenanordnung kann eine Vibrationseinrichtung umfassen. Es kann eine Vorrichtung bzw. ein System gebildet werden, welche bzw. welches die Elektrodenanordnung und eine Vibrationseinrichtung umfassen kann. Die Vibrationseinrichtung kann einen Vibrationsmotor umfassen, der in zumindest einem Teilbereich der Elektrodenanordnung angeordnet ist. Unter der Anordnung in einem Teilbereich der Elektrodenanordnung ist erfindungsgemäß zu verstehen, dass der Vibrationsmotor über, auf und an einem Teilbereich der Elektrodenanordnung angeordnet sein kann. Beispielsweise kann der Vibrationsmotor an oder auf einem Teilbereich der Elektrodenanordnung angeordnet sein und den Teilbereich der Elektrodenanordnung vibrieren. Bevorzugt kann der Vibrationsmotor mit der Elektrodenanordnung oder einem Teilbereich hiervon, insbesondere dem Elektrodenfeld, verbunden sein. Es kann auch vorgesehen sein, dass der Vibrationsmotor beabstandet zur Elektrodenanordnung angeordnet ist, um die Haut und/oder knöcherne Struktur eines Menschen oder einer Tieres zu vibrieren.

Die Positionen der Elektroden des Elektrodenfelds können so gewählt werden, dass diese der Form des Körpers, an der das Elektrodenfeld in Anlage gelangen soll, entspricht. Es ist möglich, dass die Verteilung der Elektroden des Elektrodenfelds der Form eines zur Ausübung eines Druckes vorgesehenen Druckkörpers folgt. Beispielsweise können für unterschiedliche Stellen am Körper unterschiedliche Druckkörper vorgesehen sein. Es sind beispielsweise als Pelotten ausgestaltete Druckkörper für ein Knie oder ein Schultergelenk bekannt. Die Elektroden des Elektrodenfelds können flächig über der Fläche des Druckkörpers verteilt sein.

Es kann eine Vorrichtung zur Behandlung von Schmerzen eines Menschen oder eines Tieres geschaffen werden. Die Vorrichtung weist eine Elektrodenanordnung, die ein Elektrodenfeld und mindestens eine hierzu beabstandete weitere Elektrode umfasst, und einen Druckkörper auf, wobei der Druckkörper so ausgestaltet ist, dass mindestens Teile der Elektrodenanordnung an den Körper des Menschen oder des Tieres gedrängt werden können (Ausführungsform 1). Die genannte Ausführungsform 1 kann insbesondere wie folgt weitergebildet werden:
2. Ausführungsform: Vorrichtung gemäß Ausführungsform 1, wobei der Druckkörper elastisch und inkompressibel ausgestaltet ist.
3. Ausführungsform: Vorrichtung gemäß Ausführungsform 1 oder 2, wobei ein Tragelement den Druckkörper zumindest teilweise umgibt.
4. Ausführungsform: Vorrichtung gemäß Ausführungsform 3, wobei das Tragelement als Band und/oder anatomisch angepasst geformtes Gewirk ausgestaltet ist.
5. Ausführungsform: Vorrichtung gemäß Ausführungsform 3 oder 4, wobei das Tragelement elastisch und/oder dehnbar ist.
6. Ausführungsform: Vorrichtung gemäß einer der Ausführungsformen 3 bis 5, wobei das Tragelement mittels eines Klettverschlusses schließbar ist.
7. Ausführungsform: Vorrichtung gemäß einer der Ausführungsformen 1 bis 6, wobei der Druckkörper als Pelotte ausgebildet ist, die vorzugsweise einen elastischen Werkstoff, beispielsweise Silikonkautschuk, Polyurethan oder einen ähnlichen Werkstoff, aufweist.
8. Ausführungsform: Vorrichtung gemäß Ausführungsform 7, wobei der Druckkörper eine Kniegelenkspelotte ist, deren Projektionsfläche mindestens 90cm², insbesondere mindestens 100cm², und insbesondere bevorzugt mindestens 110 cm² beträgt.
9. Ausführungsform: Vorrichtung gemäß Ausführungsform 8, wobei die Kniegelenkspelotte eine ringförmige oder halbringförmige Basis zum Umgreifen der Patella sowie einen medialen und einen lateralen Abschnitt aufweist, die asymmetrisch zueinander ausgestaltet sind, wobei die Kniegelenkspelotte eine ellipsenförmige Ausnehmung aufweist, deren kleiner Radius r ist, und der mediale Abschnitt drei Vorsprünge aufweist, von denen sich ein erster Vorsprung in distaler Richtung und ein weiterer, zweiter Vorsprung in proximaler Richtung erstrecken, wobei der Umfang der Kniegelenkspelotte zwischen dem weiteren, dritten Vorsprung eine Krümmung aufweist, die größer ist als das 2fache von r.
10. Ausführungsform: Vorrichtung gemäß Ausführungsform 9, wobei der Umfang der Kniegelenkspelotte zwischen dem zweiten Vorsprung und dem weiteren, dritten Vorsprung eine Krümmung aufweist, die größer als 2r ist.
11. Ausführungsform: Vorrichtung gemäß einer der Ausführungsformen 8 bis 10, wobei der laterale Abschnitt eine minimale Erstreckung von dem Rand der Ausnehmung bis zum Rand der Kniegelenkspelotte 1,5cm aufweist.
12. Ausführungsform: Vorrichtung gemäß einer der Ausführungsformen 8 bis 11, wobei in der Ausnehmung eine Vibrationseinrichtung vorgesehen ist, die vorzugsweise proximal in der Ausnehmung angeordnet ist.
13. Ausführungsform: Vorrichtung gemäß einer der Ausführungsformen 1 bis 6, wobei der Druckkörper als Abstandsgewirke ausgebildet ist.
14. Ausführungsform: Vorrichtung gemäß Ausführungsform 13, wobei die Form des Tragelements bandförmig und kegelstumpfförmig ist.
15. Ausführungsform: Vorrichtung gemäß Anspruch 14, wobei mindestens zwei Elektroden mit flächiger Kontaktfläche vorhanden sind.
16. Ausführungsform: Vorrichtung gemäß einer der Ausführungsformen 1 bis 15, wobei das Elektrodenfeld halbkugelförmige, punktuelle Elektroden aufweist und die weitere Elektrode eine flächige Elektrode ist.
17. Ausführungsform: Vorrichtung gemäß einer der Ausführungsformen 1 bis 16, wobei die Elektroden des Elektrodenfelds als Abschnitt einer leitfähigen Struktur auf einem nichtleitfähigen Textil ausgestaltet sind, und das Textil mit dem Tragelement unter Ausbildung eines Zwischenraums für den Druckkörper verbunden ist.

Ein Druckkörper kann als Pelotte oder als Abstandsgewirke ausgestaltet sein. Eine Pelotte kann als Stütze für den Bereich der Anlage auf der Haut, insbesondere für ein Gelenk, dienen. Eine Pelotte kann einen elastischen Werkstoff aufweisen. Eine Pelotte kann beispielsweise Silikonkautschuk, Polyurethan oder einen ähnlichen Werkstoff aufweisen oder aus diesem gefertigt sein. Der Druckkörper kann einen Anpressdruck gewährleisten. Der Druckkörper kann eine Formstabilität gewährleisten, wobei der Druckkörper zum Einen elastisch, aber auch zum Anderen inkompressibel sein kann. Im Sinne der Erfindung erfasst der Begriff Druckkörper einen Körper, der permanent einen Raum ausfüllt. Ein erfindungsgemäßer Druckkörper weist vorzugsweise einen insbesondere festen Körper, der als Festkörper ausgestaltet sein kann, auf, der eine räumliche Ausdehnung besitzt, die mindestens 60%, bevorzugt mindestens 80%, des Volumens des Druckkörpers umfasst, die vorzugsweise nur durch Ausübung von Druck auf den Körper veränderbar ist. Der Begriff Druckkörper im Sinne der Erfindung umfasst insbesondere Körper mit einer geschlossenen Hülle, deren eingeschlossenes Volumen unveränderbar ist.

Die Elektrodenanordnung bzw. Teile davon kann bzw. können auf dem Druckkörper selbst ausgestaltet sein. Es können leitfähige Strukturen zur Ausbildung der Elektrodenanordnung bzw. Teilen davon auf der Oberfläche des Druckkörpers vorgesehen sein. In einer Ausführungsform ist die Elektrodenanordnung auf einem Textil, Gewirke oder Stoff ausgebildet. Es kann vorgesehen sein, dass die Elektrodenanordnung ein leitfähiges Textil, das in einem nicht leitfähigen Textil bzw. einem anderen Material eingebettet ist, umfasst. Es kann vorgesehen sein, dass die Elektrodenanordnung leitfähige Fäden umfasst, die in einem nicht leitfähigen Textil oder anderem Material eingewirkt sind. Hierdurch wird eine einfach handhabbare Vorrichtung geschaffen, die einfach herstellbar ist.

Eine erfindungsgemäße Vorrichtung kann ein Tragelement umfassen, das den Körper eines Menschen mit dem Bereich der Haut, die die Elektrodenanordnung kontaktieren soll, zumindest teilweise umgibt. Das Tragelement kann ein Textil, eine Folie, ein Kunststoff, Leder oder ähnliches sein. Das Tragelement kann als Band oder als Strumpf oder schlauchförmig ausgestaltet sein. Das Textil kann ein Gestrick sein, das anatomisch formgestrickt ist. Das Tragelement ist vorzugsweise dehnbar und/oder elastisch.

In einer Ausführungsform kann eine erfindungsgemäße Vorrichtung eine Elektrodenanordnung, einen Druckkörper und ein Tragelement aufweisen. Die Elektrodenanordnung ist über dem Druckkörper angeordnet. Der Druckkörper kann zwischen Elektrodenanordnung und Tragelement angeordnet sein. Die auf einem Textil ausgestaltete Elektrodenanordnung kann mit dem Tragelement verbunden bzw. vernäht sein. In dem Tragelement kann eine Durchführung mit mindestens einem Leiter zur Kontaktierung der Elektrodenanordnung vorgesehen sein. Die Durchführung kann auch für die Kontaktierung einer Gegenelektrode genutzt werden.

Der Begriff Abstandsgewirke erfasst erfindungsgemäß doppelflächige Textilien, bei denen insbesondere die kettengewirkten Warenflächen durch abstandshaltende Verbindungsfäden, sogenannte Polfäden, auf Distanz gehalten werden.

Vorzugsweise können die flächig verteilt angeordneten Elektroden des Elektrodenfelds als Abschnitt einer leitfähigen Struktur auf einem nicht leitfähigen Textil ausgestaltet sein, das mit dem Tragelement unter Ausbildung eines Zwischenraums für den Druckkörper verbunden ist. Das Tragelement und das Textil können eine Tasche für den Druckkörper bilden, in dem der Druckkörper angeordnet ist und keine Befestigung zwischen Druckkörper und Textil bzw. Tragelement vorliegen muss.

Im Falle, dass der Druckkörper eine Kniegelenkspelotte ist, ist die Projektionsfläche der Kniegelenkspelotte mindestens 90cm², mindestens bevorzugt 100cm², insbesondere bevorzugt mindestens 110cm² groß. In einer bevorzugten Ausführungsform ist die Projektionsfläche der Kniegelenkspelotte etwa 102cm² groß.

Es kann für das Elektrodenfeld die vorgenannte Form eines Elektrodenarrays verwendet werden. Beispielsweise kann das Elektrodenfeld 17 Reihen und 17 Spalten von Kontaktpunkten aufweisen, die als Kathoden dienen können. Das Elektrodenfeld kann genauso wie ein Elektrodenarray bemessen und ausgestaltet bzw. realisiert werden. Insbesondere so, wie es nachfolgend für das Elektrodenarray bzw. die Igel-Elektrode beschrieben ist.

Im Falle eines Elektrodenfelds mit beabstandeter, weiterer Elektrode (Gegenelektrode) kann der mit den Elektroden überspannten Fläche eine entscheidende Rolle zukommen. So ist es wahrscheinlich, dass eine möglichst große Fläche eines nozizeptiven bzw. rezeptiven Feldes stimuliert werden muss, um eine LTD (Langzeithemmung, long term depression) optimal zu induzieren, da auch eine Mindestzahl von Afferenzen gereizt werden sollten, um diesen Mechanismus auszulösen. Grundsätzlich kann die Größe der mit Elektroden versehenen Fläche des Elektrodenfelds leichter erweitert werden, was einen technischen Vorteil beispielsweise gegenüber einer konzentrischen Elektrodenanordnung darstellt.

Sofern der Begriff Elektrodenanordnung verwendet wird, so sind hierunter im Sinne der Erfindung eine konzentrische Anordnung, ein Elektrodenfeld mit beabstandeter, weiterer Elektrode (Gegenelektrode) und ein Elektrodenarray gleichermaßen begrifflich erfasst.

Zur Auslösung von EPSPs (exzitatorisches postsynaptisches Potenzial, excitatory post-synaptic potential) an Hautafferenzen kann eine erfindungsgemäße Elektrodenanordnungen als Oberflächenelektrode verwendet werden. Durch Aufbringen der Elektrodenanordnung auf der Hautoberfläche wird durch eine elektrische Pulsform, die mittels Frequenz, Pulsbreite und Pulsamplitude (Stromstärke) charakterisiert sein kann, zwischen Anode und Kathode ein elektrisches Feld aufgebaut. Durch dieses elektrische Feld können Afferenzen, die sich in den oberflächlichen Hautschichten befinden, stimuliert werden. Das nozizeptive System besitzt verschiedene Arten von Schmerzfasern, die mit Hilfe der Elektrostimulation beeinflusst werden können. Aδ-Fasern, entsprechen den Faserstrukturen für die Verarbeitung schneller akuter Schmerzen, wobei die C-Fasern den dumpfen Schmerz vermitteln. Die verschiedenen Nervenfasern transportieren dabei die Reizmodalitäten. Die einzelnen Nervenfasern werden nachfolgend noch näher beschrieben.

Es kann vorgesehen sein, dass die Elektrodenanordnung eine Vibrationseinrichtung umfasst, die einen Vibrationsmotor aufweisen kann. Der Vibrationsmotor kann mittels Steckverbindung mit einer Steuereinheit, die auch die Elektrodenanordnung mit elektrischen Impulsen bzw. Pulsen beaufschlagt, verbunden und ansteuerbar sein. Bei dem Vibrationsmotor kann es sich beispielsweise um einen PicoVibe™-Vibrationsmotor handeln. Besonders bevorzugt ist ein Vibrationsmotor mit einer Nennspannung von 1,5V. Besonders bevorzugt ist eine typische Vibrationsamplitude von 1,2G. Es kann vorgesehen sein, dass die typische Leistungsaufnahme 150mW sind und ein Betriebsstrom von etwa 100mA vorliegt. Der Vibrationsmotor kann an einem Teilbereich der Elektrodenanordnung befestigt sein und diese vibrieren lassen. Der Vibrationsmotor kann mittelbar in mechanischem Kontakt zu einem Teilbereich der Elektrodenanordnung stehen. Beispielsweise kann der Vibrationsmotor an einer Pelotte befestigt sein, die in mechanischem Kontakt zu einem Teilbereich der Elektrodenanordnung steht.

Es hat sich herausgestellt, dass die erfindungsgemäße Elektrodenanordnung bzw. Vorrichtung, insbesondere mit Elektrodenanordnung mit Elektrodenfeld und Gegenelektrode, vor allem bei Gonarthrose, Phantomschmerz, Tiefenschmerz, oberflächlichem Schmerz bzw. Hautirritation (Juckreiz, Neurodermitis), neuropathischer Schmerz und/oder Tumorschmerz Anwendung finden kann. Es ist auch möglich, dass die Elektrodenanordnung bzw. Vorrichtung bei der Behandlung von Schmerzen in folgenden Bereichen bzw. folgenden Anwendungen und/oder Behandlungen und/oder Eingriffen am Menschen oder Tier verwendet werden: Haarentfernung, Piercing, Tattoo, Ohrringe, Hautstraffung, Entfernung von Muttermalen, Hautunebenheiten und kleineren dermatologischen Eingriffen.

Es kann vorgesehen sein, dass die Elektrodenanordnung ein leitfähiges Textil oder eine leitfähige flexible Struktur, das in einem nicht leitfähigen Textil bzw. einem anderen Material eingebettet oder auf diesem aufgebracht ist, umfasst. Vorzugsweise sind die Elektroden kugelförmig oder halbkugelförmig ausgestaltet, insbesondere die Elektroden des Elektrodenfelds. Es kann vorgesehen sein, dass die Elektrodenanordnung leitfähige Fäden umfasst, die in einem nicht leitfähigen Textil oder anderem Material eingewirkt sind. Die Elektroden des Elektrodenfelds können als Abschnitte des leitfähigen Fadens ausgebildet sein. Hierdurch wird eine einfach handhabbare Elektrodenanordnung geschaffen, die einfach herstellbar ist.

Eine Trägerstruktur kann dabei durch das nicht leitfähige Textil oder ein anderes Material geschaffen werden. Es kann auch vorgesehen sein, dass auf ein Textil oder anderes Material eine leitfähige Struktur aufgedruckt ist, die die Elektrodenanordnung bzw. einen Teilbereich der Elektrodenanordnung umfasst. Es kann auch vorgesehen sein, dass auf ein leitfähiges Material eine Struktur bzw. Fläche aufgedruckt wird, die nicht leitfähig ausgebildet ist. Bei der Struktur, die aufgedruckt wird, kann es sich um eine flächige Struktur, aber auch um eine kugelförmige Struktur handeln. Die kugelförmige Struktur kann in der Höhe eine variierende Geometrie aufweisen. Es kann auch vorgesehen sein, dass die Elektrodenanordnung mindestens eine auf ein Material oder ein Textil aufgestickte bzw. aufgebrachte Elektrode (beispielsweise in

Form einer Kugel oder eines Kegelstumpfs oder einer Spitze) aufweist.

Die genannten Beispiele ermöglichen eine einfache Integration der Elektrodenanordnung beispielsweise in eine Bandage. Die Elektrodenanordnung oder ein Teilbereich der Elektrodenanordnung kann in die Trägerstruktur integriert werden. Als Trägerstruktur sind vorzugsweise aus dem medizinischen Bereich (v.a. sogenannte orthopädische Hilfsmittel) bekannte Anordnungen möglich, wobei eine Anpassung zwischen Elektrodenanordnung und Trägerstruktur erfolgen kann. Es kann auch vorgesehen sein, dass die Elektrodenanordnung auf einer flexiblen Platine ausgestaltet ist und in einem Textil oder anderem Material eingebettet ist. In einer bevorzugten Ausführungsform kann das Textil schmutzabweisend beschichtet sein, um die Kontaktierung mit der Haut über einen längeren Zeitraum zu gewährleisten bzw. zu verbessern.

Es kann vorgesehen sein, dass das Textil eine Kälte- und/oder Wärmestimulation der Haut des menschlichen oder tierischen Körpers mittels der Elektrodenanordnung, indem diese mit Kälte und/oder Wärme beaufschlagt wird, durchführen kann. Es kann auch vorgesehen sein, dass unabhängig von der Elektrodenanordnung eine Kälte- und/oder Wärmestimulation durch Kühl- oder Heizelemente im Textil durchgeführt werden kann/können (Kälte- und/oder Wärmekissen). Eine Kälte-/Wärmestimulation wird als Verbesserung des Wirkspektrums angesehen.

Bevorzugt weist im Falle des Elektrodenarrays mit Anoden und Kathoden in Reihen und Spalten der Abstand zwischen einer Anode und einer der Anode benachbarte Kathode weniger als 3cm auf. Durch die Wahl des Abstands ist eine gezielte Stimulation bestimmter Nervenfasern möglich. Insbesondere kann es auch vorgesehen sein, dass der Abstand geringer als 0,7cm ist. Kleinere Abstände von ungefähr 0,05cm bis 0,2cm sind ebenfalls möglich. Es hat sich gezeigt, dass beispielsweise für einen Abstand von ungefähr 0,25cm gute Ergebnisse hinsichtlich einer verbesserten Schmerzreduktion und/oder Schmerzunterdrückung erzielt werden, die insbesondere die Nervenfasern der oberflächigen Hautstruktur anspricht.

Bevorzugt weisen die Anoden und/oder die Kathoden (für Elektrodenfeld und Matrixelektrode) eine kugelförmige oder halbkugelförmige Oberfläche zur Anlage auf der Haut auf, wodurch neben einem positiven Empfinden für die Haut auch die Emission des Feldes in die Haut verbessert ist. Elektroden (insbesondere des Elektrodenfelds oder der Matrixelektrode) können als sogenannte Ball Grids ausgestaltet sein. Die Anoden- und Kathodenformen können zwischen spitz, stumpf, abgerundet und eckig variiert werden.

Es sind aber auch andere Anoden- bzw. Kathodenformen möglich.

Bevorzugt können die Elektroden für Elektrodenfeld und Elektrodenarray als sogenannte Ball Grids ausgestaltet sein. Dabei kann es sich um eine Platine oder andere Grundstruktur handeln, wobei an einer Fläche viele kleine "Kugeln" aufgesetzt sind. Die Platine und/oder Grundstruktur (beispielsweise auch die Tragstruktur oder das nicht leitfähige Textil) ist bevorzugt flexibel und es ist dadurch zusammen mit der Kugelform der Elektroden eine besonders gute Hautkontaktierung möglich. Die Kugeln können zum Einen als Elektrode sowie als Übertragungsinstanz der Vibration fungieren. Der Begriff "Kugel" umfasst erfindungsgemäß auch Formen, die in Richtung der Haut spitz zulaufen. Die Elektroden können sich auch in Richtung der Haut verjüngen. Spitz zulaufende Elektroden können bevorzugt sein.

Zudem kann vorgesehen sein, dass eine Einheit zur Temperaturänderung der in Kontakt mit der menschlichen bzw. tierischen Oberfläche stehenden Elektroden vorhanden ist. Mit den Elektroden könnten dann Wärme- und/oder Kälteimpulse aufgebracht werden.

In einer bevorzugten Ausführungsform können die Anode(n) bzw. Kathoden an einer Trägerstruktur angeordnet sein, wodurch eine gute Handhabbarkeit erreicht werden kann. Unter dem Begriff "an einer Trägerstruktur" ist erfindungsgemäß vorzugsweise zu verstehen, dass die Elektrodenanordnung in oder auf bzw. an der Trägerstruktur ausgestaltet ist. Bevorzugt kann die Trägerstruktur gegenüber den Anoden und/oder Kathoden elektrisch isoliert sein, um vorbestimmbare Bedingungen für die Elektrodenanordnung zu schaffen. Bevorzugt weist die Trägerstruktur ein elektrisch nichtleitendes Material auf, mit dem eine Isolierung der Anoden und/oder Kathoden erreicht werden kann. Bevorzugt kann die Trägerstruktur ein der Haut zugewandtes Material umfassen, das saugfähig ist und von den Anoden bzw. Kathoden isoliert ist. Hierdurch können definierte Bedingungen für eine Einleitung der Impulse in die Haut geschaffen werden, indem auf der Haut vorhandener Schweiß von dem saugfähigen Material aufgenommen und/oder gebunden werden kann. In einer bevorzugten Ausführungsform ist die Trägerstruktur schichtförmig ausgebildet, um mit den einzelnen Schichten zusammenwirkende Funktionen zu erzielen.

Um eine gute Anpassung an die Hautkontur zu erzielen, kann die Trägerstruktur flexibel ausgestaltet sein. Die Elektrodenanordnung schmiegt" sich gleichsam an die gewünschte Kontur.

Bevorzugt kann es auch vorgesehen sein, dass die Anoden und/oder Kathoden einen einstellbaren bzw. variierbaren Abstand zueinander bzw. untereinander aufweisen. Es kann beispielsweise vorgesehen sein, dass die Elektrodenanordnung als verschiebbare Gitterelektrode zur Einstellung der Abstände bei Verkleinerung und Vergrößerung der Trägerstrukturfläche ausgestaltet ist.

In einer bevorzugten Ausführungsform weist die Elektrodenanordnung eine Steuereinheit auf, mit der die Anoden zusammen mit Kathoden mit elektrischen Impulsen beaufschlagbar sind. Die Steuereinheit kann körperfern angebracht sein; es kann eine Übertragung der Energie bzw. Steuerimpulse durch einen Wireless-Standard erfolgen (Bluetooth, Zigbee usw.). Ferner bevorzugt können die Anoden getrennt voneinander mit einer oder mehreren Kathoden von der Steuereinheit mit elektrischen Impulsen beaufschlagbar sein, was die Flexibilität und Parametrisierung der Elektrodenanordnung und der mit ihr erzielbaren Impulseinleitungsmöglichkeiten erhöht.

Bevorzugt sind die Kathoden und/oder Anoden mit einem durch die Steuereinheit regelbaren Strom von 0,1 bis 30mA, oder höher, impulsförmig beaufschlagbar. Es kann für den regelbaren Strom bevorzugt ein Wert im Bereich von 1mA bis 20mA gewählt werden, insbesondere bevorzugt ein Wert aus dem Bereich von 1mA bis 15mA. Bevorzugt können monopolare Rechteckimpulse durch die Steuereinheit erzeugt werden, die einen besonders steilen Anstieg und einen besonders steilen Abfall aufweisen können. Der steile Anstieg und der steile Abfall können derart charakterisiert sein, dass innerhalb von 10µs die maximale Ausgangsspannung erreicht wird. Die Pulsdauer kann 50µs bis 2000µs, insbesondere 100µs bis 500µs betragen. Als Pulsfolgefrequenz kann ein Wert zwischen 0,1 Hz bis 10Hz, insbesondere zwischen 3 und 4Hz, vorzugsweise insbesondere im Wesentlichen 4Hz, gewählt werden. Es kann auch eine Pulsfolgefrequenz mit einem Wert zwischen 50Hz bis 100Hz gewählt werden, was eine "Burst-Stimulation", d.h. eine Erst-Stimulation bedeuten kann. Vorzugsweise kann der Burst-Stimulation eine Pulsfolgefrequenz mit einem Wert zwischen 0,1 Hz bis 10Hz folgen. Als bevorzugter Wert für die Pulsfolgefrequenz kann ein Wert aus dem Bereich von 0,01 Hz bis 5Hz verwendet werden, bei dem das Wirkspektrum sich als herausragend gezeigt hat. Als Spannungsversorgung der Steuereinheit ist vorzugsweise ein Akkumulator oder eine Batterie möglich, die eine maximale Spannung im zweistelligen Volt-Bereich benötigt; insbesondere bevorzugt wird ein Akkumulator bzw. wiederaufladbare Batterie oder eine Batterie verwendet, die eine maximale Spannung von ungefähr 9 Volt liefert.

Insbesondere kann vorgesehen sein, dass die Regelung des Stromes zur Beaufschlagung der Elektroden auf einen Konstantwert unter Berücksichtigung der Impulsbreite erfolgt. Hierdurch kann eine bestimmte Energie appliziert werden.

Die Steuereinheit kann als anwendungsspezifische integrierte Schaltung (ASIC) oder programmierbarer Mikroprozessor oder Kontroller ausgestaltet sein. Mit der Steuereinheit ist eine Beaufschlagung der Elektroden je nach Einstellung möglich. Sofern die Steuereinheit als Mess- und Regeleinheit ausgebildet ist, kann in Abhängigkeit von einem gemessenen bzw. erfassten Signal, beispielsweise den Hautwiderstand, eine Regelung für die Parameter der Beaufschlagung der Elektroden durchgeführt werden.

Über die Steuereinheit kann nicht nur die Pulsbreite, die Pulswiederholrate und die Pulsintensität gesteuert bzw. festgelegt werden, sondern auch die Anwendungsdauer. Beispielsweise kann ein Therapieplan oder Anwendungsregime mittels der Steuereinheit umgesetzt werden, indem die Dauer (Anwendungsdauer) vorgegeben wird, in der die Impulsbeaufschlagung erfolgt. Beispielsweise kann eine Anwendungsdauer so gewählt werden, dass die Impulsbeaufschlagung im Bereich von 1 min/h bis 20min/h erfolgt. Die Steuereinheit ist vorzugsweise programmierbar. Es sind gleich lange und/oder unterschiedlich lange Anwendungsdauern möglich, zwischen denen gleich lange und unterschiedlich lange Ruhepausen einstellbar sind.

Zudem ist über die Steuereinheit möglich, eine Erinnerungsfunktion an den Träger der Elektrodenanordnung oder der Vorrichtung zu senden, welches eine Patientencompliance verbessern kann. Ferner ist additiv oder alternativ möglich, eine Protokolldatei zu erstellen, mit der der durchgeführte Therapieplan objektiv ausgewertet werden kann (sogenanntes log-File).

In einer bevorzugten Ausführungsform ist die Elektrodenanordnung von einem Haltemittel oder Tragelement am tierischen oder menschlichen Körper anordenbar bzw. platzierbar. Die Begriffe Haltemittel und Tragelement können austauschbar verwendet werden. Das Haltemittel kann beispielsweise ein Pflaster, ein antistatisch beschichtetes Material oder eine Bandage sein. Das Haltemittel kann als Haltevorrichtung ausgestaltet sein, bei dem die Elektrodenanordnung als abtrennbare Einheit ausgebildet ist, die eine elektrische Verbindung zum Verbinden der Elektrodenanordnung mit einer an der Haltevorrichtung befindlichen Steuereinheit aufweist. Auch kann vorgesehen sein, dass die Elektrodenanordnung Teil der Haltemittel ist, aber eine Steuereinheit und/oder eine Spannungsversorgung nicht Teil der Haltemittel sind. Hierdurch kann die Möglichkeit geschaffen werden, dass die Elektrodenanordnung, die anwendungsgemäß in Kontakt mit der Haut kommt, nach einer gewissen Nutzdauer einfach ausgetauscht werden kann. Zudem kann die Möglichkeit geschaffen werden, die Haltevorrichtung ohne die Elektrodenanordnung zu reinigen. Die Steuereinheit und die Stromversorgung können in einem Gehäuse geschützt vorliegen, das beispielsweise durch eine Spritzgusstechnik herstellbar ist. Die Elektrodenanordnung stellt quasi eine Anordnung mit begrenzter oder spezifischer Nutzdauer dar, die abhängig von der Anzahl der Anwendungen auf der Haut und/oder von der Anzahl möglicher Reinigungszyklen der Elektrodenanordnung sein kann. Unter einer elektrischen Verbindung im Sinne der Erfindung kann jede lösbare Verbindung verstanden werden, die eine schnelle und sichere Verbindung zwischen Elektrodenanordnung und Steuereinheit ermöglicht, vorzugsweise ohne mechanisches Werkzeug. Mit erfasst von dem Begriff Verbindung sollen Schnappverbindungen, Steckverbindungen, Klettverbindungen, Verbindungen durch Aufschieben und Schraubverbindungen sein. Die Haltevorrichtung und die Elektrodenanordnung können so ausgestaltet sein, dass die Haltevorrichtung mit der Elektrodenanordnung beim Tragen am menschlichen oder tierischen Körper keine Bewegungseinschränkung und/oder Einschränkung hinsichtlich einer Zugänglichkeit an den Körper darstellt. Die Haltevorrichtung zum Positionieren der Elektrodenanordnung am menschlichen oder tierischen Körper kann vorzugsweise eine Dicke von weniger als 10cm, insbesondere bevorzugt eine Dicke von weniger als 5cm und ganz besonders bevorzugt eine Dicke von weniger als 3cm aufweisen.

Erfindungsgemäß wird auch eine Verwendung vorgeschlagen, bei der eine Elektrodenanordnung zur Stimulation von Nervenfasen eines Menschen oder Tieres zur Unterdrückung oder Reduzierung eines chronischen oder akuten Schmerzes verwendet wird, wobei die Elektrodenanordnung in Anlage auf die Haut des Menschen oder Tieres bringbar ist und mehrere Anoden bei der Elektrodenanordnung vorgesehen sind.

Es hat sich gezeigt, dass die vorgenannte Elektrodenanordnung insbesondere zum Erreichen oder Auslösen einer LTD an Synapsen von Nervenzellen zur Reduzierung von akuten und chronischen Schmerzzuständen ausgelöst werden kann.

Es hat sich gezeigt, dass zum Erreichen oder Auslösen einer LTD die TENS nicht ausreichend sein kann. Es soll daher auch eine Vorrichtung und ein Verfahren vorgeschlagen werden, mit dem eine LTD an Synapsen von Nervenzellen zur Reduzierung von akuten und chronischen Schmerzzuständen ausgelöst werden kann.

Es wird somit auch eine Vorrichtung zur Anwendung an der Oberfläche eines menschlichen oder tierischen Körpers mit einer Elektrodenanordnung und ein Verfahren zur Behandlung, insbesondere Reduktion, von Gonarthrose, Phantomschmerz, Tiefenschmerz, oberflächlichem Schmerz bzw. Hautirritation (Juckreiz, Neurodermitis), neuropathischer Schmerz und/oder Tumorschmerz an einem menschlichen oder tierischen Körper mit einer Elektrodenanordnung vorgeschlagen. Eine Ausführungsform der Erfindung ist eine Vorrichtung zur Induzierung einer dauerhaften Abschwächung der Signalübertragung an den Synapsen von Nervenzellen (Langzeitdepression) am menschlichen oder tierischen Körper mit Hilfe der elektromechanischen Schmerzsuppression (EMSS). Dadurch können akute oder chronische Schmerzzustände, sowie Juckreize reduziert werden. Der Mechanismus kann des Weiteren als elektromechanisches Lokalanästhetikum bei Kleinsteingriffen oder Wundschmerz genutzt werden.

Das nozizeptive System hat zwei Arten von Schmerzfasern: Aδ- und C-Nervenfasern. Bei den Aδ-Fasern handelt es sich um relative dicke (ca. 3-5µm) und aufgrund der Ummantelung durch eine sog. Myelinscheide, schnell leitende (5 - 50m/s) Fasertypen. Hingegen sind die C-Fasern dünn (ca. 1µm) und nicht myelinisiert. Sie zeigen Reizleitungsgeschwindigkeiten von unter 1 m/s. Aδ-Fasern enden auf Rückenmarksebene in den Schichten I und V des Hinterhorns, wo sie Synapsen mit Projektionsneuronen bilden. Die meisten C-Fasern hingegen sind mit Interneuronen in Schicht II verschaltet. In den Schichten III und IV des Hinterhorns enden die Axone der Berührungs- und Temperatursensoren.

Die beiden Populationen von Schmerzfasern erzeugen eine zeitliche Verzögerung in der Schmerzempfindung: Der erste Schmerz, der in Bruchteilen einer Sekunde wahrgenommen wird, wird durch die Aδ-Fasern geleitet und ist oft stechend oder brennend. Sekunden später trifft der durch C-Fasern vermittelte Schmerz ein, der als bohrend oder dumpf beschrieben wird.

Aus der transkutanen elektrischen Nervenstimulation (TENS) ist bekannt, eine elektromedizinische Reizstromtherapie mit Rechteckimpulsen niedriger Frequenz, d.h. 2 bis 4Hz, oder hoher Frequenz, d.h. 80 bis 100Hz, anzuwenden. Die TENS wird vor allem zur Behandlung von Schmerzen und zur Muskelstimulation eingesetzt. Über Elektroden werden elektrische Impulse auf die Oberfläche eines menschlichen Körpers oder tierischen Körpers übertragen. Meist erfolgt die Platzierung der Elektroden in der Nähe der schmerzenden Stellen.

Kommt es zu einer dauerhaften Reizung der oben beschriebenen zwei Arten von Schmerzfasern, so entsteht auf Rückenmarksebene eine sog. Langzeitpotenzierung (longterm potentiation (LTP)). Durch Experimente konnte nachgewiesen werden, dass die Langzeitpotenzierung durch frequenzspezifische Stimulation der afferenten Nervenfasern verschwindet. Eine Langzeithemmung bzw. Langzeitdepression (longterm depression (LTD)) der synaptischen Übertragung kann durch niederfrequente elektrische Stimulation hervorgerufen werden. Insbesondere zur Reduktion von chronischen Schmerzen soll der neurobiologische Schmerzmechanismus einer Sensibilisierung zentraler Schmerzneurone im Hinterhorn des Rückenmarks (zentrale Sensibilisierung) durch eine elektrische Reizung als "Gegenirritation" (LTD) reguliert werden. Ein weiterer wichtiger Effekt ist die voraussichtliche Stimulation/Förderung des absteigenden schmerzhemmenden Systems (dieses unterdrückt das aufsteigende schmerzwahrnehmende System). Bei der zentralen Sensibilisierung der Schmerzverarbeitung handelt es sich um einen bei allen möglichen Schmerzerkrankungen vorkommenden krankhaften Mechanismus (Schmerzgedächtnis). Demgegenüber handelt es sich bei der LTD um einen u.a. spinalen, im Rückenmark lokalisierten Lernmechanismus, der zu einer Regulation der Schmerzempfindlichkeit nach unten führt, d.h. dass eine zuvor gesteigerte Schmerzempfindlichkeit, z.B. im Rahmen eines Rückenschmerzes und/oder einer Gonarthrose, wieder reduziert wird. Es wird also erfindungsgemäß das Schmerzgedächtnis beeinflusst und dem erlernten Schmerz entgegengewirkt. So wird eine verbesserte Mobilität, Alltagsbelastbarkeit und damit erhöhte Lebensqualität erreicht.

Es hat sich herausgestellt, dass erfindungsgemäß eine bevorzugte Ausführungsform zur Schmerzreduktion bzw. Schmerzunterdrückung eine Elektrodenanordnung ist, die über eine Vibrationseinrichtung zum Vibrieren gebracht wird, wobei die Übertragung von periodischen, mittel- bis höherfrequenten und niederamplitudigen Schwingungen auf die Hautoberfläche ermöglicht wird, wobei durch die Elektrode die Hautoberfläche mechanisch so gespannt wird, dass eine optimale Schwingfähigkeit erreicht wird. Dadurch können verschiedene Nervenfasern (zum Beispiel Aß-Fasern) gereizt werden, die wiederum Aδ- und C-Fasern in ihrer Schmerzweiterleitung (modifizierte Gate-Control-Hypothese) unterbrechen können.

In einer bevorzugten Ausführungsform wird die Elektrodenanordnung und/oder die Haut und/oder knöcherne Struktur des Menschen oder Tieres im Bereich zumindest eines Teils der Elektrodenanordnung vibriert, wodurch überwiegend markhaltige Mechanoafferenzen (Aß-Fasern) erregt werden. Unter dem hier verwendeten Begriff Bereich wird erfindungsgemäß die nähere Umgebung und/oder der von zumindest einem Teil der Elektrodenanordnung überdeckte Bereich der Haut erfasst.

Erfindungsgemäß wird unter "Anlage zur Oberfläche" verstanden, dass die Elektrodenanordnung im Wesentlichen auf der Haut des Menschen bzw. des Tieres aufliegt. Es kann aber auch insbesondere bei einer Wunde vorgesehen sein, dass die Elektrodenanordnung zur Wundversorgung um die Wunde herum in Anlage zu der die Wunde umgebenden Haut gelangt.

Durch spezifisch angepasste Stimulationsmuster kommt es auf mehreren Ebenen des schmerzverarbeitenden Systems zu Veränderungen an den spinalen nozizeptiven Informationseingängen. Die Substantia gelatinosa im Hinterhorn des Rückenmarks übernimmt die Funktion eines "Tores" für eingehende nozizeptive Informationen. Aβ-Fasern können hemmende Effekte auf die im Hinterhorn eingehenden Impulse der Aδ- und C-Fasern haben. Dadurch kommt es zu einer Modulation an der Substantia gelatinosa, was zu einer Aktivierung der inhibitorischen Interneurone führt. Bei einem normalen Schmerzreiz wird eine Aktivierung der exzitatorischen Interneurone an der Substantia gelantinosa ausgelöst. Durch verschiedene Umschaltmechanismen entsteht schließlich der typische Schmerz.

Durch verschiedene Impulsmuster zwischen 0,1 und 20Hz, insbesondere 1 bis 4Hz, kann der oben beschriebene Effekt der LTD ausgelöst werden. Er unterscheidet sich grundlegend von früheren/verwandten Ansätzen basierend auf der TENS-Technologie (transkutane elektrische Nervenstimulation). Durch die betrachteten Elektrodenanordnungen wird eine nur oberflächliche Reizung der Cutis und Subcutis mit den in diesen Schichten verlaufenden Aδ- und C-Nervenfasern erreicht. Neben den speziellen Elektroden ist dazu eine integrierte Mess- und Regeltechnik notwendig, die situations- und patientenspezifisch eine Parameterjustierung gewährleistet. Der für die Stimulierung eines myelinisierten Nervs durch Hautelektroden notwendige Strom hat bevorzugt einen monophasischen rechtwinkligen Impuls in einer Dauer von ungefähr 0,05 bis 3ms, die variiert werden kann.

Abgesehen von der Amplitude ist die Impulsbreite mitentscheidend für das Faserspektrum, das erregt wird. Je kürzer der Impuls, desto höher muss die Amplitude sein und umgekehrt. Um die zur Schmerzlinderung benötigten großkalibrigen Fasern zu erregen, wird bevorzugt eine ausreichende, meist fixierte Impulsbreite, üblicherweise ungefähr 0,2 bis 0,3ms, verwendet, um die Voraussetzung zu schaffen, dass die Amplitude reduziert werden kann. Als Impulsform wird vorzugsweise ein Rechteck-Impuls verwendet.

Es kann auch vorgesehen sein, dass die Impulsform einen sinusförmigen Verlauf aufweist.

Die Elektrodenanordnung kann ein Elektrodenarray sein. Unter einem Elektrodenarray kann eine Anordnung von Elektroden verstanden werden, die flächenmäßig ausgestaltet ist. Es sind mehrere Elektroden vorgesehen in einer Fläche zwischen ungefähr einem und mehreren Quadratmillimetern. So sind beispielsweise Flächen zwischen 1 cm x 1 cm bis 10cm x 10cm möglich. Eine quadratische Fläche kann verwendet werden, allerdings sind auch rechteckige Flächen oder kurvig umrandete Flächen erfindungsgemäß erfasst. Insbesondere bevorzugt ist, dass die flächenförmige Anordnung der Elektroden flexibel hinsichtlich der Oberfläche der Elektroden, die in Anlage mit der Oberfläche des menschlichen oder tierischen Körpers gelangt, gestaltet ist, wodurch die Elektrodenanordnung auf die Oberfläche eines menschlichen oder tierischen Körpers ohne Kontaktverlust aufgebracht werden kann. Die Anordnung der Elektroden in dem Elektrodenarray ist derart, dass Anoden und Kathoden in eine Linie alternierend zueinander benachbart in dem Elektrodenarray angeordnet sind. In der anderen Linie des Elektrodenarrays sind gleichartige Elektroden, d.h. Anoden bzw. Kathoden, nebeneinander angeordnet. Vorzugsweise ist der Abstand zwischen einer Kathode und einer benachbarten Anode im Bereich von 1,5mm bis 3,5mm, insbesondere bevorzugt liegt ein Abstand von 2,5mm zwischen direkt benachbarter Anode und Kathode vor. Die Kontaktflächen können möglichst kurz gewählt werden, sodass eine Impulsapplikation unter dem Sekretionsfilm der Haut aufgebracht werden kann. Dadurch wird eine effektive Reizung der in der Cutis und Subcutis verlaufenden Nervenfasern erreicht. Eine Muskelstimulation wird aufgrund der kurzen Stromflüsse zwischen den Anoden und den hierzu benachbarten Kathoden jeweils nahezu ausgeschlossen.

An einer Fläche können viele kleine Elektroden aufgesetzt sein. Die Fläche ist bevorzugt flexibel und es ist dadurch zusammen mit der Form der Elektroden eine besonders gute Hautkontaktierung möglich. Auch wenn der Vibrationsmotor nicht direkt in Kontakt mit der Elektrodenanordnung ist (sondern die umliegende Haut und/oder knöcherne Struktur vibriert), kann sich ein positiver Effekt über die Elektrodenform einstellen, dadurch dass zumindest ein Teilbereich der Elektrodenanordnung, der an der Haut anliegt, zumindest mittelbar vibriert wird. Insbesondere kann als Elektrodenform ein spitz zulaufender Kegel, eine spitz zulaufende Kugel oder eine spitz zulaufende oder sich verjüngende andere Form verwendet werden.

Zudem kann vorgesehen sein, dass eine Einheit zur Temperaturänderung der Ball Grids bzw. der in Kontakt mit der menschlichen bzw. tierischen Oberfläche stehenden Elektrodenflächen vorhanden ist. Mit den Elektroden bzw. den Ball Grids könnten dann Wärme- und/oder Kälteimpulse aufgebracht werden. Zudem können die Ball Grids als Messköpfe einer Mess- und Regeleinheit ausgestaltet sein, mit denen ein Hautwiderstand erfasst werden kann. Durch eine automatische Variierung der Impulsstärke kann somit ein optimaler Effekt erzielt werden.

Es kann auch vorgesehen sein, dass die Elektrodenanordnung mindestens eine konzentrische Elektrode aufweist, die eine, vorzugsweise schmal ausgestaltete, zentrale Kathode und eine die Kathode umgebende größere, vorzugsweise ringförmig ausgestaltete, Anode umfasst. Es kann auch vorgesehen sein, dass die Kathode in viele kleine zentrisch angeordnete Kathoden aufgespaltet ist. Wichtig ist auch hier, dass der Abstand der beiden Elektroden zueinander nicht größer als 3,5mm ist, insbesondere bevorzugt kleiner als 2,5mm ist. Es kann auch vorgesehen sein, dass die Anode zentral ausgebildet ist und die Kathode, die Anode umgibt. Die Elektroden können flexibel oder massiv angefertigt werden. Bevorzugt sind die Anode und die Kathode ringförmig ausgestaltet, wobei die Kathode einen Durchmesser im Bereich von wenigen Millimetern aufweist, bevorzugt ist der Durchmesser 1 mm. Die Anode weist bevorzugt einen inneren Durchmesser von ungefähr 8mm und einen äußeren Durchmesser von etwa 24mm auf.

Es kann aber auch vorgesehen sein, dass nur eine Elektrode vorgesehen ist und gegen eine Referenzelektrode abgeleitet wird.

In einer bevorzugten Ausführungsform weist die Vorrichtung eine Mess- und Regeleinheit auf, die in den Stimulationspausen eine Erfassung des aktuellen Hautwiderstandes bzw. der Impedanz ermöglicht. Durch eine Feedbackschleife kann auf Grundlage des erfassten aktuellen Hautwiderstandes bzw. der Impedanz der durch die Elektrodenanordnung ausgesendete Impuls angepasst werden. Die Mess- und Regeleinheit erfasst damit den aktuellen Hautwiderstand bzw. die Impedanz und verwendet den erfassten Wert als Regelgröße zur Ansteuerung der Elektrodenanordnung. Es kann damit der Sekretionsstatus der Haut ermittelt und als Regelgröße verwendet werden.

Vorzugsweise sind die Leiterbahnen der Elektrodenanordnungen bzw. die Elektrodenanordnung flexibel ausgestaltet oder weisen Gelelektroden und/oder Textilelektroden auf. Es kann somit vorgesehen sein, dass eine Gelmatrix die Elektrodenanordnung umfasst, die entweder selbst elastisch ist oder aus elastisch verbundenen Einzelteilen oder Bausteinen besteht, in der alle Leitungsbahnen teilweise oder komplett durch ein leitendes Medium (Flüssigkeit, Elektrolyt, metallischer Leiter) aufgebaut sind. Damit wird die Elektrode wie ein Gelkissen oder eine Blisterverpackung konstruiert. Die komprimierbare Füllung erlaubt einen besonders guten Kontakt zur Haut. Durch die Vibrationseinrichtung zur Vibration der Elektrodenanordnung können Vibrationen über das Flüssigmedium besonders gut direkt auf die Hautoberfläche übertragen werden. Im Falle, dass die Elektrodenanordnung Textilelektroden aufweist, wird ein leitendes Textil verwendet, in der alle Leitungsbahnen entweder aufgedruckt oder als leitendes Material eingewebt werden. Diese Umsetzung erlaubt einen besonders guten Tragekomfort sowie eine gute Kontaktierung auf der Hautoberfläche. Ferner wird durch das Textil, für das beispielsweise Neopren verwendet werden kann, eine besonders gute Isolierung der Körperstelle erreicht. Hautsekretionen, d.h. Schweiß, können über das Textil gut resorbiert werden, sodass das Risiko von Kurzschlüssen zwischen Anode und Kathode verringert wird. Es kann aber auch vorgesehen sein, dass die Elektrodenanordnung massive Leiterbahnen aufweist. Die Kontaktelemente, d.h. die äußere Oberfläche der Elektrodenanordnung, die der Oberfläche eines menschlichen bzw. tierischen Körpers zugewandt ist, kann federnd gelagert sein gegenüber der massiven Leiterbahn. Dies kann beispielsweise durch einen Federmechanismus ausgestaltet sein, durch den die Kontakte, die so genannten Pins, auf einer Feder bzw. Federanordnung sitzend, sich in eine Matrix hinein drücken lassen. Hierdurch kann die Elektrodenanordnung auch auf gekrümmte Flächen eines menschlichen bzw. tierischen Körpers aufgesetzt werden.

Es kann vorgesehen sein, dass die Elektrodenanordnung mit einer Beschichtung aus einer pharmakologischen Substanzklasse versehen ist, wodurch neben der Stimulation ein zusätzlicher Effekt, beispielsweise zur Wundheilung, erreichbar ist. Die Beschichtung erfolgt derart, dass die mit der menschlichen bzw. tierischen Oberfläche in Kontakt gelangenden Elektroden der Elektrodenanordnung endseitig beschichtet sind. Die Substanzklassen können beispielsweise Lokalanästhetika, kortisonhaltige Substanzen usw. sein.

In einer bevorzugten Ausführungsform kann die Stromversorgung der Vorrichtung durch Energy Harvesting erfolgen. Dazu kann eine Einrichtung zur Erzeugung von Energie vorgesehen sein, die mit der Elektrodenanordnung verbunden ist. Das primäre Ziel beim Energy Harvesting im Bezug auf die vorliegende Anmeldung ist es, die Pulserzeugungseinheit zum Beaufschlagen der Elektrodenanordnung mit Spannung und die Vibrationseinrichtung zur Vibration der Elektrodenanordnung effektiv mit Energie zu betreiben. Die zum Betreiben der Vorrichtung erforderliche Energie kann zumindest teilweise beispielsweise durch die Ausnutzung der Temperaturdifferenz zwischen Haut und Umgebung gewonnen werden. Es ist auch möglich, dass die kinetische Energie ausgenutzt wird, die z. B. durch eine Bewegung des Anwenders erzeugt wird. Für die Ausnutzung der kinetischen Energie kann eine Implementierung der Vorrichtung in Bandagen, Orthesen oder Stützeinheiten erforderlich sein. Die kinetische Energie kann beispielsweise mechanisch - ähnlich dem Aufzugmechanismus automatischer Uhren - erfolgen. Das Aufladen kann kabellos über Induktion erfolgen. Es kann vorgesehen sein, dass die Vorrichtung einen Akkumulator bzw. eine wiederaufladbare Batterie umfasst, die über Energy Harvesting aufgeladen wird. Der Akkumulator bzw. die wiederaufladbare Batterie kann mit der Pulsgeneratoreinheit und der Vibrationseinheit zur Energieversorgung verbunden sein.

Bevorzugt ist die Vorrichtung - und insbesondere der Teilbereich der Vorrichtung mit der Pulserzeugungseinheit - gegen Schweißeinwirkung und Waschvorgänge geschützt, da die Vorrichtung zum Einsatz in Bandagen, Band etc. kommen kann, die gewaschen werden müssen, und die Vorrichtung aufgrund des Anwendungsgebietes beim Einsatz Feuchtigkeit, Schmutz, Chemikalien und/oder Krafteinwirkung ausgesetzt ist. Es kann bevorzugt sein, dass die Schaltung zum Beaufschlagen der Elektrodenanordnung fest verschweißt ist. In einer bevorzugten Ausführungsform ist eine Polsterung - zum Beispiel in Form eines Gelkissens - vorgesehen, die Krafteinwirkungen auf die Schaltung abpuffert bzw. reduziert.

Die Erfindung kann ferner ein Verfahren zur Reduktion von chronischen Schmerzen, akuten Schmerzen wie beispielsweise Juckreizen, Inzisions- und/oder Wundschmerz an einem menschlichen oder tierischen Körper mit einer Elektrodenanordnung schaffen. Es wird eine oberflächliche Stimulation der Cutis und Subcutis durch die Elektrodenanordnung durchgeführt. Die Elektrodenanordnung kann bei der Durchführung der oberflächlichen Stimulation vibriert werden.

Bevorzugt wird bei dem Verfahren die Schweißsekretion ermittelt, um die durch die Elektrodenanordnung ausgesendeten Pulse über die Pulserzeugungseinheit mittels einer Mess- und Regeleinheit zu regeln.

Bevorzugt wird Energie gewonnen zum Beaufschlagen der Elektrodenanordnung. Das Gewinnen der Energie erfolgt derart, dass eine benachbart zur Elektrodenanordnung vorgesehene Umwandlungseinheit vorgesehen ist, die mechanische oder thermische Energie in elektrische Energie umwandelt. Die elektrische Energie kann in einem Akkumulator bzw. einer wiederaufladbaren Batterie zwischengespeichert werden.

In einer bevorzugten Ausführungsform wird eine Stimulation über einen längeren Zeitraum als eine Minute mit einer Frequenz von 0,1 bis 20Hz ausgelöst.

Nachfolgend wird die Erfindung an Hand von Zeichnungen näher erläutert, die allerdings lediglich mögliche Ausführungsformen der Erfindung zeigen. Darin zeigen:
- Fig. 1:: eine schematische Darstellung einer erfindungsgemäßen Elektrodenanordnung,
- Fig. 2:: eine schematische Darstellung einer erfindungsgemäßen Vorrichtung,
- Fig. 3a:: eine schematische Draufsicht auf eine Elektrodenanordnung der Vorrichtung gemäß Fig. 2,
- Fig. 3b:: die Elektrodenanordnung der Fig. 3a in einer schematischen perspektivischen Ansicht,
- Fig. 4:: eine alternative Ausführungsform der Elektrodenanordnung der Vorrichtung gemäß Fig. 2,
- Fig. 5:: mechanische Schmerzschwelle bei 1 Hz,
- Fig. 6:: mechanische Schmerzschwelle bei 3Hz,
- Fig. 7:: mechanische Schmerzschwelle bei 4Hz,
- Fig. 8:: Druckschmerzschwelle (PPT),
- Fig. 9:: eine Igelelektrode,
- Fig. 10:: ein Funktionsmuster,
- Fig. 11:: Testareale,
- Fig. 12:: Verlauf der Schmerzschwelle bei BASELINE (Tag 1,2), SHAM (Tag 3, 4) und EMSS (Tag 5, 6),
- Fig. 13:: PPT-Ergebnisse Testareal 1 (medial),
- Fig. 14:: PPT-Ergebnisse Testareal 2 (Patella),
- Fig. 15:: PPT-Ergebnisse Testareal 3 (lateral)
- Fig. 16:: schematische Darstellung einer Verteilung der Stromdichte in mA/cm² und der Stromlinienverläufe für zwei flächige Elektroden,
- Fig. 17:: schematische Darstellung einer Verteilung der Stromdichte in mA/cm² und Stromlinienverläufe für eine Matrixanordnung mit einer Gegenelektrode,
- Fig. 18:: schematische Darstellung einer Verteilung der Stromdichte in mA/cm² und Stromlinienverläufe für eine konzentrische Elektrodenanordnung,
- Fig. 19:: schematische Darstellung einer Elektrodenanordnung für das Knie,
- Fig. 20:: schematische Darstellung eines Druckkörpers für die Elektrodenanordnung von Fig. 19 mit einer Vibrationseinheit,
- Fig. 21a:: schematische Seitenansicht in einer "Explosionsdarstellung" der Elektrodenanordnung von Fig. 19 mit dem Druckkörper von Fig. 20 und einem Tragelement sowie einem Knie,
- Fig. 21b:: schematische Vorderansicht der Darstellung von Fig. 21a,
- Fig. 22a:: schematische Darstellung eines Tragelements/Bandes mit einer Elektrodenanordnung in einer Ansicht auf die Elektrodenanordnung,
- Fig. 22b:: schematische Darstellung des Tragelements/Bandes von Fig. 22a in einer Schnittansicht von oben,
- Fig. 23:: eine Tabelle mit einer Varianzanalyse (ANOVA) der Studie,
- Fig. 24:: eine Übersicht von QST(quantitative sensorische Testung)-Daten,
- Fig. 25:: einen Vergleich zwischen Matrixelektrode bzw. Elektrodenanordnung mit Elektrodenfeld und beabstandeter Elektrode und konzentrischer Elektrode nach 4Hz Stimulation,
- Fig. 26:: einen Vergleich zwischen Matrixelektrode bzw. Elektrodenanordnung mit Elektrodenfeld und beabstandeter Elektrode und konzentrischer Elektrode nach 30Hz Stimulation,
- Fig. 27:: eine Faktorenanalyse,
- Fig. 28a:: synaptische Langzeitdepression (LTD) mit normaler Schmerzwahrnehmung,
- Fig. 28b:: synaptische Langzeitdepression (LTD) mit einer elektrischen niederfrequenten kutanen Stimulation,
- Fig. 28c:: synaptische Langzeitdepression (LTD) nach niederfrequenter Stimulation,
- Fig. 29:: elektrisches Modell der Haut,
- Fig. 30a:: schematische Darstellung einer zentralen Sensibilisierung,
- Fig. 30b:: schematische Darstellung einer Normalisierung der Sensibilität durch LTD; und
- Fig. 31:: eine grafische Darstellung von Zwei-Punkt-Diskriminations-Tests.

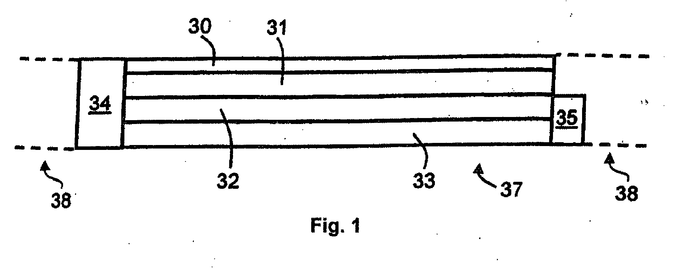

In Fig. 1 ist schematisch eine erfindungsgemäße Elektrodenanordnung dargestellt. Es ist eine Elektrodenanordnung mit Elektroden 30 dargestellt, die in Anlage mit der Haut eines Menschen oder eines Tieres gelangen kann. Die Elektroden 30 sind auf oder in einer Trägerstruktur 31 angeordnet, die vorzugsweise allergenfrei ausgestaltet ist. Die Trägerstruktur 31 kann auch Noppen aus Silikon bzw. mit Silikon beschichtete Noppen aufweisen. Auf der die Elektroden 30 aufweisenden gegenüberliegenden Seite der Trägerstruktur 31, ist die Trägerstruktur 31 in mechanischer Verbindung mit einem mechanischen Applikator, der in Form eines Vibrationsmotors 32 ausgestaltet ist. Der Vibrationsmotor 32 wird als Schwingungsgenerator verwendet, um eine Vibration auf die Haut über die Elektroden 30 und/oder die Noppen auf der Trägerstruktur 31 zu applizieren. Durch die Trägerstruktur 31 und mit ihr mechanisch verbundenen weiteren Elementen kann die Vibration auf eine große Fläche übertragen werden. Rückseitig des Vibrationsmotors 32 ist eine flexible Außenschicht 33 vorgesehen. Die Elektroden 30, die Trägerstruktur 31, der Vibrationsmotor 32 und die Außenschicht 33 bilden eine Einheit 37, die als Ganzes gehandhabt werden kann.

Zum Betreiben der Elektrodenanordnung bzw. Elektroden 30 und des Vibrationsmotors 32 ist eine Spannungsquelle bzw. Energieversorgung 34 vorgesehen, die mit den Elektroden 30 und dem Vibrationsmotor 32 elektrisch in Form einer Steckverbindung verbunden werden kann. Die Elektrodenanordnung bzw. die Elektroden 30 und/oder der Vibrationsmotor 32 können über eine als Mess- und Regelelektronik ausgestaltete Steuereinheit 35 gesteuert werden. Die Steuereinheit 35 kann die Elektroden 30 der Elektrodenanordnung mit Pulsen beaufschlagen.

Die Einheit 37 kann in eine Haltevorrichtung 38, die schematisch mit gestrichelten Linien dargestellt ist, eingesetzt werden. Die Steuereinheit 35 und die Energieversorgung 34 können Teil der Haltevorrichtung 38 sein. Vorzugsweise kann die Energieversorgung 34 lösbar mit der Haltevorrichtung 38 verbunden sein, um beispielsweise im Falle eines Defekts die Energieversorgung 34 austauschen zu können. Die Haltevorrichtung 38 kann eine (Aktiv-)Bandage oder eine (aktiv-)bandagenartige Ausgestaltung aufweisen.

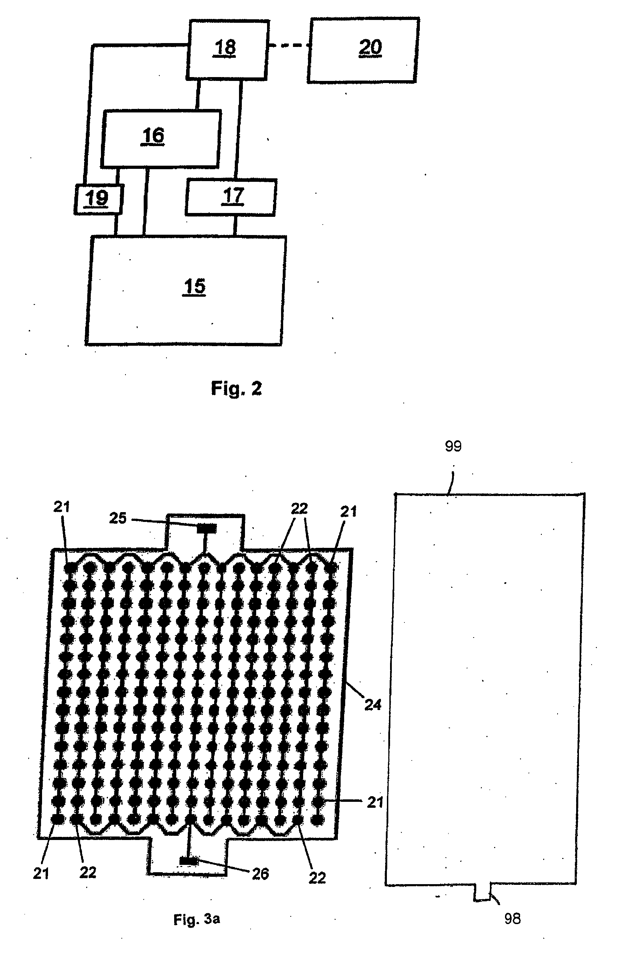

In Fig. 2 ist schematisch eine erfindungsgemäße Vorrichtung gezeigt. Die Vorrichtung ist zur Anwendung an der Oberfläche eines menschlichen Körpers vorgesehen und weist dazu eine Elektrodenanordnung 15 auf, die in Anlage zur Oberfläche des menschlichen Körpers gelangen kann. Die Oberfläche der Elektrodenanordnung 15 umfasst Enden von Elektroden. Es ist eine Pulserzeugungseinheit 16 mit der Elektrodenanordnung operativ verbunden. Über die Pulserzeugungseinheit 16 kann die Elektrodenanordnung 15 mit Spannung bzw. Strom beaufschlagt werden. Ferner weist die Vorrichtung eine Vibrationseinrichtung 17 zur Vibration der Elektrodenanordnung 15 auf. Die Vibrationseinrichtung 17 kann in der Elektrodenanordnung 15 körperlich ausgebildet bzw. körperlich mit dieser verbunden sein. Zur Energieversorgung der Pulserzeugungseinheit 16 und der Vibrationseinrichtung 17 ist eine Einrichtung 18 zur Bereitstellung von Energie vorgesehen, die mit der Pulserzeugungseinheit 16 und der Vibrationseinrichtung 17 verbunden ist. Die Einrichtung 18 zur Bereitstellung von Energie, die als Akkumulator bzw. wiederaufladbare Batterie ausgestaltet sein kann, versorgt die Pulserzeugungseinheit 16 und die Vibrationseinrichtung 17 mit Strom bzw. Spannung.

Die Vorrichtung weist gemäß Fig. 2 ferner eine Mess- und Regeleinheit 19 auf, mit der beispielsweise an der Elektrodenanordnung 15 der aktuelle Hautwiderstand bzw. die Impedanz ermittelt werden kann. Der von der Mess- und Regeleinheit 19 erfasste Wert kann als Regelungsgröße verwendet werden. Die Mess- und Regeleinheit 19 kann auf die mit ihr verbundene Pulserzeugungseinheit 16 Einfluss nehmen derart, dass in Abhängigkeit von der Größe des erfasste Messwerts die Dauer der Impulse und/oder die Abstände zwischen den Impulsen geregelt bzw. verändert werden. Auch die Mess- und Regeleinheit ist zur Strom- bzw. Spannungsversorgung mit der Einrichtung 18 zur Bereitstellung von Energie verbunden.

Ferner ist in Fig. 2 eine Einrichtung 20 zur Umwandlung von thermischer und/oder mechanischer Energie in elektrische Energie als Teil der Vorrichtung in einem Ausführungsbeispiel gezeigt, mit der die Einrichtung 18 zur Bereitstellung von Energie mit elektrischer Energie versorgt werden kann. Durch die gestrichelte Linie zwischen der Einrichtung 18 zur Bereitstellung von Energie und der Umwandlungseinrichtung 20 ist angedeutet, dass die Verbindung auch kabellos, d.h. in diesem Falle induktiv, erfolgen kann.

In Fig. 3a ist schematisch eine Draufsicht auf die Elektrodenanordnung 15 der Vorrichtung gemäß Fig. 2 in einem ersten Ausführungsbeispiel gezeigt. Die Ansicht ist derart, dass auf die die Oberfläche des menschlichen bzw. tierischen Körpers gelangende Oberfläche der Elektrodenanordnung 15 geschaut wird. Die Elektrodenanordnung 15 umfasst kugelförmige Elektrodenenden an einer Platine 24, die als Kathoden 21, 22 ausgestaltet sind. Die Platine 24 ist flexibel. Die Anordnung der Kathoden 21, 22 ist flächig in Form eines Elektrodenarrays. Eine Anode 99 ist flächig, beabstandet zu den Kathoden 21, 22 vorgesehen. In einer Linie des Elektrodenarrays, die um 90° zur ersten Linie sich erstreckt, sind gleichartige Elektroden nebeneinander angeordnet, die miteinander elektrisch leitfähig verbunden sind. Zum Anschluss der Kathoden 21, 22 und der Anode 99 ist ein Anodenanschluss 98 und ein Kathodenanschluss 25, 26 vorgesehen. Die Kathodenanschlüsse 25, 26 sind an einem Fortsatz der Platine 24 außerhalb der Fläche der Elektrodenanordnung vorhanden.

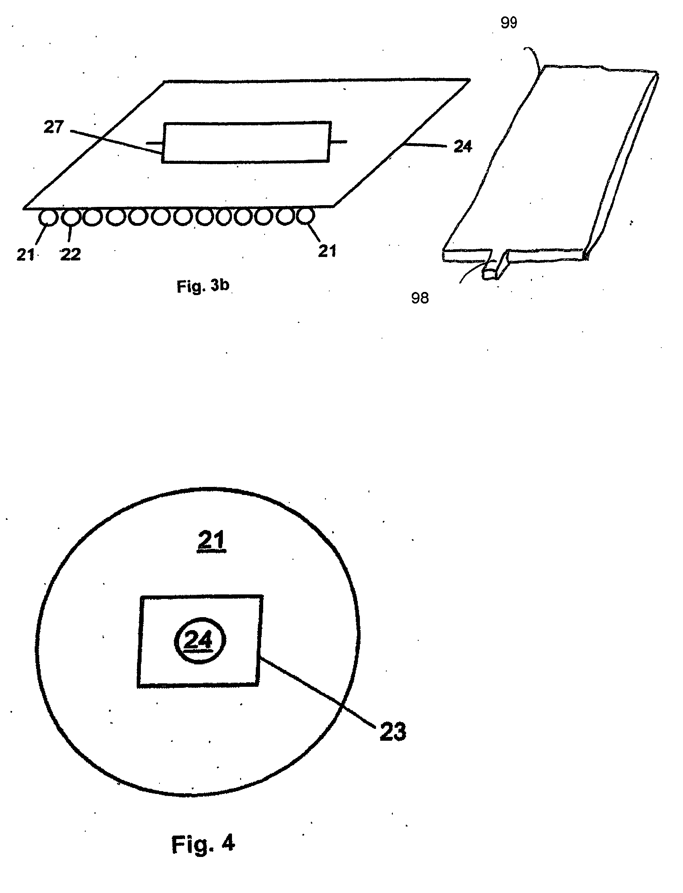

In Fig. 3b ist schematisch die Elektrodenanordnung 15 gemäß Fig. 3a in einer perspektivischen Ansicht dargestellt. Die Enden der Kathoden 21, 22 der Elektrodenanordnung liegen in Form von Kugeln vor. Es ist ein Vibrationsmotor 27 gezeigt, der die Platine 24 mit den Kathoden 21, 22 kontaktiert. Die Kontaktierung des Vibrationsmotors 27 mit der Platine 24 kann derart sein, dass die Längsenden des Vibrationsmotors 27 jeweils mit der Platine 24 verbunden sind. Die Anode 99 liegt flächig auf der Haut auf.

In Fig. 4 ist eine alternative Ausführungsform der Elektrodenanordnung 15 der Vorrichtung gemäß Fig. 2 gezeigt. Eine ringförmige schmale zentrale Kathode 22 ist innerhalb einer ebenfalls kreisförmig ausgestalteten Anode 21 ausgebildet. Die endseitigen Oberflächen der Anode 21 und der Kathode 22 können in Kontakt mit der Oberfläche des menschlichen Körpers gelangen. Zwischen der Anode 21 und der Kathode 22 ist eine Aufnahme 23 für die Kathode 22 angeordnet, die mit der Vibrationseinrichtung 17 mechanisch verbunden ist zur Vibration der Elektrodenanordnung 15. Die ringförmig ausgestaltete Anode 21 weist einen inneren Durchmesser von 8mm und einen äußeren Durchmesser von 24mm auf. Die schmale zentrale Kathode 22 weist einen Durchmesser von 1 mm auf.

Die Erfindung ist vorstehend anhand einiger Ausführungsformen beschrieben worden. Der Fachmann erkennt, dass Überschneidungen und Kombinationen der Ausführungsformen gewollt und beabsichtigt sein können. Beispielsweise können die in den Fig. 1 bis 4 dargestellten und beschriebenen Ausführungsformen kombiniert werden. Parametereinstellungen zur Beaufschlagung der Elektroden - beispielsweise Pulsdauer, Frequenz, Amplitude usw., die hinsichtlich einer Ausführungsform beschrieben sind, können auch für Ausführungsformen verwendet werden, für die diese nicht explizit genannt sind. Ferner ist es möglich, die Elektrodenanordnung erfindungsgemäß mittels verschiedenartiger Haltemittel, d.h. vorzugsweise mittels eines (Wund-)Pflasters, eines antistatisch beschichteten Materials (wobei das antistatisch beschichtete Material auch als schmutzabweisende Beschichtung ausgestaltet sein kann), einer Bandage oder einem bandförmigen Textil usw., auf der Haut anzuordnen bzw. zu platzieren. Die Elektrodenanordnung kann auch Teil der Haltevorrichtung sein.

Eine erfindungsgemäße Elektrodenanordnung kann vorzugsweise eine flächige Anordnung von Elektroden, eine konzentrisch um eine zentral angeordnete Elektrode angeordnete Elektrode oder eine Kombination aufweisen. Alle Elektrodenanordnungen können bevorzugt eine Masse von unter 500g, weiter bevorzugt unter 100g, insbesondere bevorzugt unter 20g, aufweisen. Die Elektrodenanordnung kann zur Auflage mit der Haut ausgestaltet sein. Es kann auch vorgesehen sein, dass die Elektrodenanordnung Elektroden aufweist, die die Haut durchdringen bzw. perforieren. Die flächige Anordnung von Elektroden, die konzentrisch um eine zentral angeordnete Elektrode angeordnete Elektrode oder eine Kombination hiervon können so ausgestaltet sein, dass der Abstand zwischen einer Anode und einer der Anode benachbarten Kathode weniger als 3cm beträgt, wobei auch oben angegebene Werte möglich sind. Elektroden aller zuvor genannten Elektrodenanordnungen können auf einer Platine ausgebildet sein, die flexibel ausgestaltet ist. Die Elektroden der flächigen Elektrodenanordnung, der konzentrischen Elektrodenanordnung und Mischformen hiervon können eine halbkugelförmige Oberfläche zur Anlage auf der Haut aufweisen. Alle zuvor genannten Elektrodenanordnungen können an einer Trägerstruktur angeordnet sein. Die an einer Platine ausgebildeten Elektroden, die als Ball Grids ausgestaltet sein können, können in eine Trägerstruktur eingebracht bzw. an dieser angeordnet sein. Die Erhebung der Elektroden von der Trägerstruktur kann größer als der Schweißfilm auf der Haut ausgebildet sein. Die Trägestruktur kann ein elektrisch nichtleitendes Material umfassen, mittels dem die Elektroden gegenüber der Trägerstruktur isoliert sind. Die Trägerstruktur von flächiger Elektrodenanordnung, konzentrischer Elektrodenanordnung oder Kombination der beiden Elektrodenanordnungen kann ein der Haut zugewandtes Material umfassen, das saugfähig ist. Für alle Elektrodenanordnungen kann die Trägerstruktur schichtförmig angeordnete Materialien umfassen. Die Trägerstruktur kann für alle Elektrodenanordnungen flexibel ausgestaltet sein. Der Abstand der Elektroden aller Elektrodenanordnungen kann einstellbar sein. Die Elektrodenanordnungen können von einer Steuereinheit mit elektrischen Impulsen beaufschlagbar sein. Die Steuereinheit kann an der Trägerstruktur oder körperfern ausgebildet sein. Die Elektroden aller Elektrodenanordnungen können impulsförmig beaufschlagbar sein, wobei vorzugsweise ein regelbarer Strom von 1 bis 15mA verwendet wird. Als Pulsdauer kann für alle Elektrodenanordnungen eine Dauer von 100µs bis 500µs verwendet werden. Für alle Elektrodenanordnungen kann eine Pulsfolgefrequenz von 0,1 Hz bis 30Hz verwendet werden. Als Erst-Stimulation (Burst-Stimulation) ist eine andere Pulsfolgefrequenz möglich. Als Spannungsversorgung für die Elektrodenanordnungen kann ein Akkumulator oder eine Batterie verwendet werden, der bzw. die eine Spannung von 9V zu Verfügung stellt. Für die Elektrodenanordnungen kann als Steuereinheit ein programmierbarer Mikroprozessor vorgesehen sein. Für die Elektrodenanordnung, d.h. die flächige Elektrodenanordnung, die konzentrische Elektrodenanordnung und Mischformen hiervon, kann ein Haltemittel vorgesehen sein. Das Haltemittel kann als Textil, Bandage, antistatisch beschichtetes Material usw. ausgestaltet sein. In dem Haltemittel kann die Steuereinheit für die Elektrodenanordnungen, fest oder lösbar, ausgebildet sein. Die Elektrodenanordnung kann fest oder lösbar mit dem Haltemittel verbunden bzw. in dem Haltemittel integriert vorliegen.

Alle Elektrodenanordnungen können mittels eines Vibrationsmotors in Schwingungen bzw. Vibration versetzt werden. Dazu kann die Elektrodenanordnung mit einem Vibrationsmotor mechanisch verbunden sein. Es wurde festgestellt, dass eine Vibrationseinheit eine angenehme niederfrequente Stimulation erlaubt, stimuliert aber darüber hinaus vor allem eine weitere Nervenfaserart (Aß-Fasern) die den bereits oben genannten Wirkmechanismus verstärkt. Eine niederfrequente Stimulation bewirkt eine schwache Aktivierung von NMDA-Synapsen und führt nur zu einem leichten Anstieg der Kalzium-Konzentration intrazellulär. Dies bewirkt über einen entsprechenden Zellmechanismus eine Langzeit-Depression (LTD). Das so genannte NMDA-Rezeptor-abhängige LTD (NMDAR-LTD) und metabotrope Glutamat-Rezeptor-abhängige LTD (mGluR-LTD) sind die bisher bekannten Mechanismen die angesprochen werden können. Bei NMDAR-LTD wird der so genannte NMDA-Rezeptor durch eine gleichzeitige Transmitterausschüttung an der Synapse und eine elektrische Erregung aktiviert, so dass Kalzium in die Zelle einströmen kann. Als intrazelluläres Signalmolekül aktiviert Kalzium auch bei LTD eine Reihe weiterer Enzyme, die die Signalübertragung an der Synapse regulieren. Bei mGluR-LTD werden metabotrope Glutamatrezeptoren aktiviert, die als so genannte G-Proteine über weitere Signalmoleküle die Ausschüttung von Kalzium aus intrazellulären Speichern auslösen. Beide LTD-Typen können an denselben Synapsen vorkommen, nutzen aber getrennte Mechanismen. Es konnte festgestellt werden, dass über eine Langzeitstimulation z.B. mit Hilfe einer Bandage das Schmerzgedächtnis beeinflusst werden kann. Es kann durch oben genannte Mechanismen zu einer Reduktion bzw. Normalisierung von krankhaften Schmerzzuständen kommen.

Die Steuereinheit kann den Vibrationsmotor mit Strom bzw. Spannung versorgen. Die Steuereinheit kann für alle Elektrodenanordnungen als Mess- und Regeleinheit ausgestaltet sein, mit der vorzugsweise die Schweißsekretion ermittelt werden kann. Die Elektroden der Elektrodenanordnungen können Gelelektroden oder Textilelektroden umfassen. Mindestens eine der Elektroden der Elektrodenanordnungen kann mit einer Beschichtung aus einer pharmakologischen Substanzklasse versehen sein. Für jede der vorgenannten Elektrodenanordnungen kann Energie zum Beaufschlagen der Elektrodenanordnungen gewonnen werden.

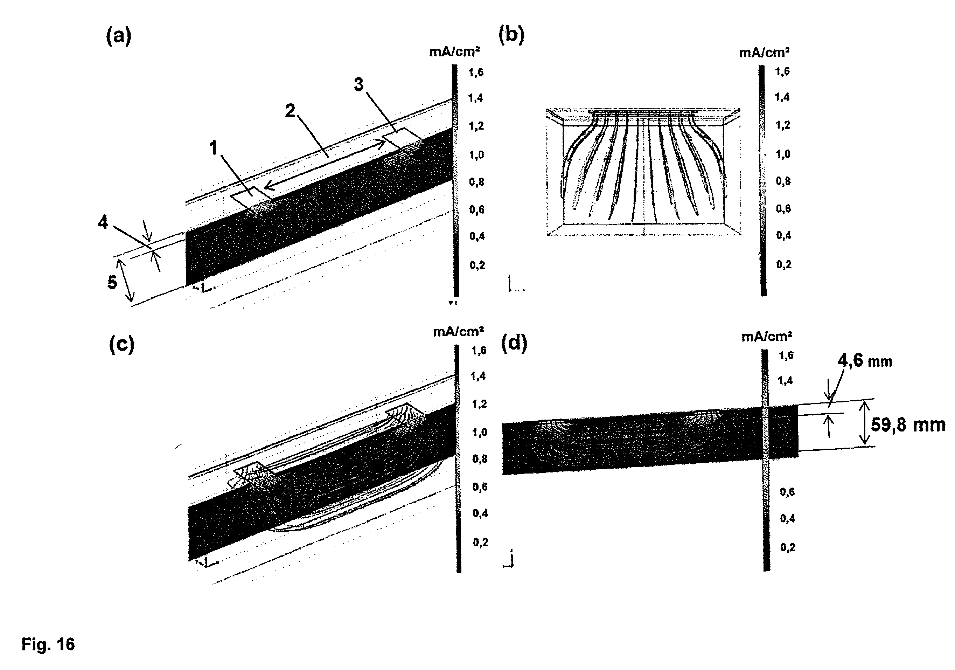

In Fig. 16 ist allgemein eine schematische Darstellung zur Verteilung der Stromdichte in mA/cm² und der Stromlinienverläufe zweier flächiger Elektroden als Kathode und Anode gezeigt, die sich aus einer FEM-Simulation ergibt. Angenommen wurde ein Elektrodenabstand von 200mm [2]. Fig. 16(a) ist eine Übersicht der Stromdichteverteilung unter der Kathode [1] und Anode [3]. Dabei zeigt [5] die maximale Eindringtiefe von 59,8mm und [4] die Eindringtiefe mit der höchsten Stromdichte von 4,6mm hier 1 mA. Fig. 16(b) ist eine Betrachtung der Stromdichte und der Stromlinien im Querschnitt. Fig. 16(c) ist eine Übersicht der Stromdichteverteilung und der Stromlinien von Kathode zu Anode. Fig. 16(d) ist ein Längsschnitt der Stromdichteverteilung und der Stromlinien von Kathode zu Anode.

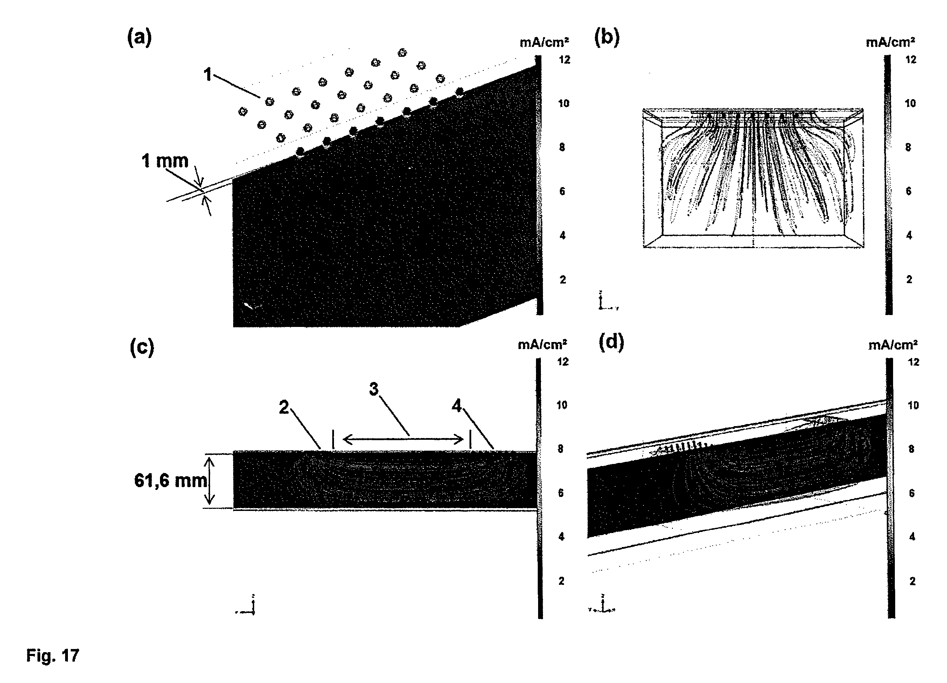

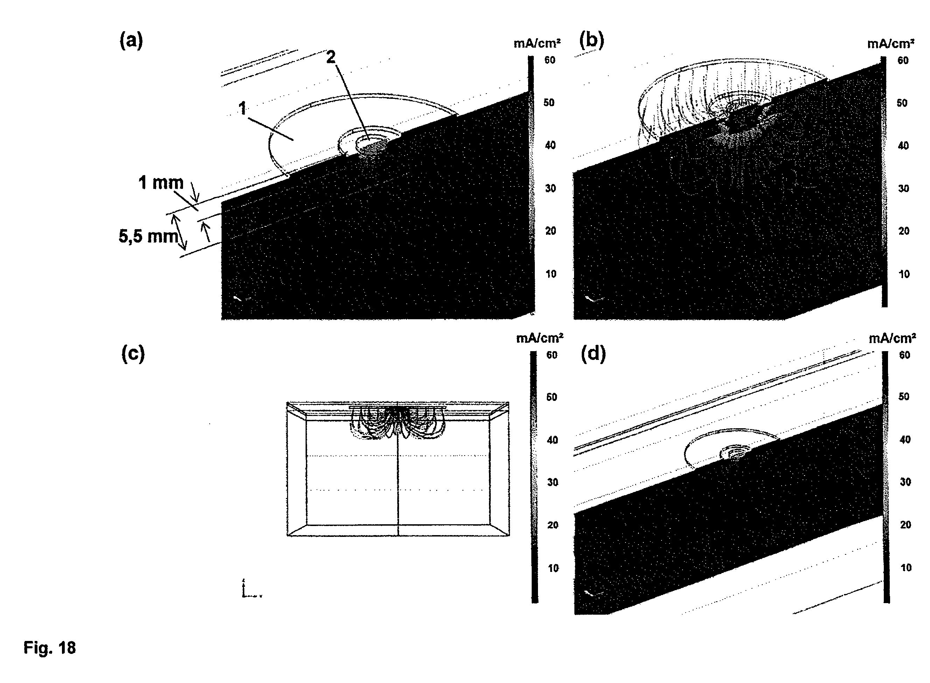

Bei der konzentrischen Elektrode beträgt der Anoden-Kathodenabstand nur wenige Millimeter, so dass die Eindringtiefe (Fig. 18) des elektrischen Stromes in die Haut gering ist. Bei der Matrixelektrode bzw. dem Elektrodenfeld ist der Anoden-Kathodenabstand deutlich größer (Fig. 17). Durch die punktuelle Kontaktierung der Kathodenfläche entstehen im Vergleich zu der flächigen Gelelektrode (Fig. 16) lokal hohe Stromdichten (Fig. 18), die primär in den oberen Hautschichten abgeleitet werden. Da die nozizeptiven freien Nervenendigungen in den oberflächlichen Hautarealen zu finden sind, können diese somit durch die Stimulation erfasst werden.

In den nachfolgenden Abschnitten werden die Matrixelektrode sowie die konzentrische Elektrode am Beispiel von FEM-Simulationen näher erläutert.

Bei einem Elektrodenfeld handelt es sich um ein dreidimensionales Multielektrodenarray. Durch die Beschichtung mit sog. Ball-Grids (Kugelgitteranordnung) wird eine punktuelle Kontaktierung auf der Haut gewährleistet (Fig. 17).

Fig. 17 ist eine schematische Darstellung einer FEM-Simulation allgemein für ein Elektrodenfeld [1]: Verteilung der Stromdichte in mA/cm² und Stromlinienverläufe. Der Abstand [3] zwischen Elektrodenfeld und zweiter Elektrode (Referenzelektrode) [4] beträgt 200mm. Durch die Ball-Grid-Beschichtung [1] wird eine punktuelle Stromdichte in den oberflächlichen Hautarealen erreicht. Die Stromdichten mit 12mA/cm² werden dabei in einer Tiefe von 1 mm (Ort der primären Hautafferenzen) erreicht. Die maximale Eindringtiefe kann mit 61,6mm dargestellt werden. (a) Stromdichteverteilung unter dem Elektrodenfeld. (b) Betrachtung der Stromdichte- und Stromlinienverteilung zwischen Elektrodenfeld und weiterer Elektrode im Querschnitt. (c) Längsschnitt der Stromlinien von Kathode zu Anode. (d) Längsschnitt der Stromdichteverteilung und der Stromlinien von Kathode zu Anode.

In Fig. 18 ist eine schematische Darstellung einer FEM-Simulation gezeigt: Verteilung der Stromdichte und Stromlinienverläufe der konzentrischen Elektrode. Die konzentrische Elektrode (Anode [1] und zentrale Kathode [2]) besitzt in einer Tiefe von 1 mm noch eine Stromdichte von 20mA/cm². Die maximale Eindringtiefe kann bei diesem Elektrodenmodell mit 5,5mm beschrieben werden. (a) Stromdichteverteilung unter der konzentrischen Elektrode. (b) Betrachtung der Stromdichte- und Stromlinienverteilung zwischen Anode und Kathode. (c) Querschnitt der Stromlinien von Kathode zu Anode. (d) Längsschnitt der Konzentrischen Elektrode.

Die konzentrische Elektrodenanordnung besteht aus einer großen Ring-Anode mit einem inneren Durchmesser von 8mm und einem äußeren Durchmesser von 24mm. Im Zentrum der Elektrode befindet sich eine zentrale Kathode mit einem Durchmesser von 1 mm. Auf Grund des konzentrischen Designs wird bereits durch niedrige Stromstärken eine hohe Felddichte erreicht. Allerdings ist die Größe des stimulierten Hautareals deutlich reduziert (Fig.18).

Dadurch kann die oberflächliche Hautschicht, in der sich freie Nozizeptoren befinden stimuliert werden.

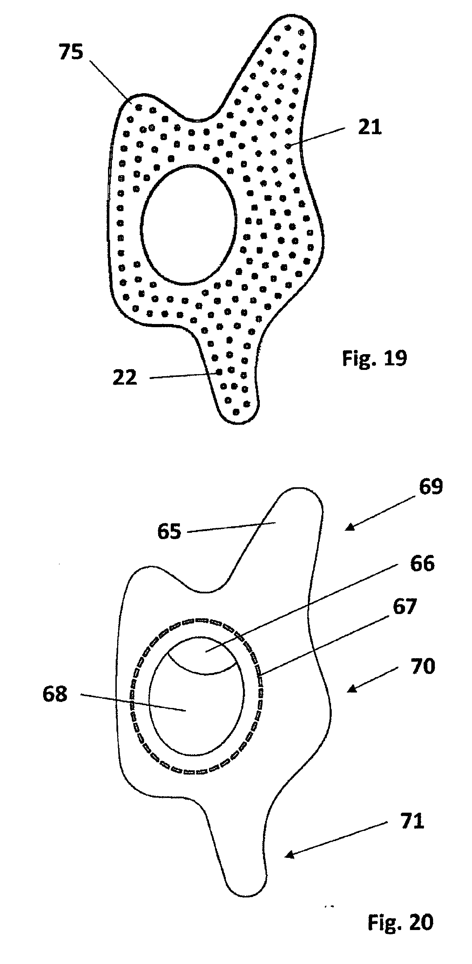

In Fig. 19 ist schematisch eine Elektrodenanordnung für ein Knie gezeigt. Die Elektroden 21, 22 sind flächig verteilt und für eine punktuelle Auflage auf der Haut ausgestaltet. Die Elektroden 21, 22 sind in bzw. auf einem Textil 75 ausgestaltet. Die Elektroden 21, 22 bilden punktuelle Erhöhungen auf dem Textil 75.

In Fig. 20 ist schematisch eine Kniegelenkspelotte als Druckkörper 65 für die in Fig. 19 gezeigte Elektrodenanordnung für das Knie gezeigt. An dem Druckkörper 65 ist eine Vibrationseinheit 66 angeordnet. Die als Vibrationsmotor ausgestaltete Vibrationseinheit 66 ist an der Pelotte befestigt. Die Vibrationseinheit 66 ist so angeordnet, dass die Vibrationseinheit 66 über und/oder an (mit Haut bedeckten) knöchernen Strukturen des Knies anliegen kann. Beispielsweise kann die Vibrationseinheit direkt an der Haut anliegen oder über einem Textil oder anderem Material mit der Haut in Kontakt sein. Die knöchernen Strukturen können als Resonanzkörper dienen.

Die in Fig. 20 gezeigte Kniegelenkspelotte weist eine ringförmige Basis zum Umgreifen der Patella auf. Die ringförmige Basis ist durch eine gestrichelte Linie 67 und die Ausnehmung 68 in der Basis visualisiert. Die Ausnehmung 68 weist im Wesentlichen eine elliptische Form auf, wobei r der kleine Radius der Ellipse ist. Die Kniegelenkspelotte weist einen medialen und einen lateralen Abschnitt auf, die asymmetrisch zueinander ausgestaltet sind. Der mediale Abschnitt weist drei Vorsprünge 69, 70, 71 auf, von denen sich ein erster Vorsprung 71 in distaler Richtung und ein weiterer, zweiter Vorsprung 69 in proximaler Richtung erstrecken. Der weitere, dritte Vorsprung 70 erstreckt sich zwischen den beiden Vorsprüngen 69, 71. Der Radius der Krümmung des äußeren Umfangs der Kniegelenkspelotte ist zwischen dem ersten Vorsprung 71 und dem weiteren, dritten Vorsprung 70 nicht größer als 3r, insbesondere nicht größer als 2r. Der Radius der Krümmung des äußeren Umfangs der Kniegelenkspelotte ist zwischen dem zweiten Vorsprung 69 und dem weiteren, dritten Vorsprung 70 nicht größer als 3r, insbesondere nicht größer als 2r.

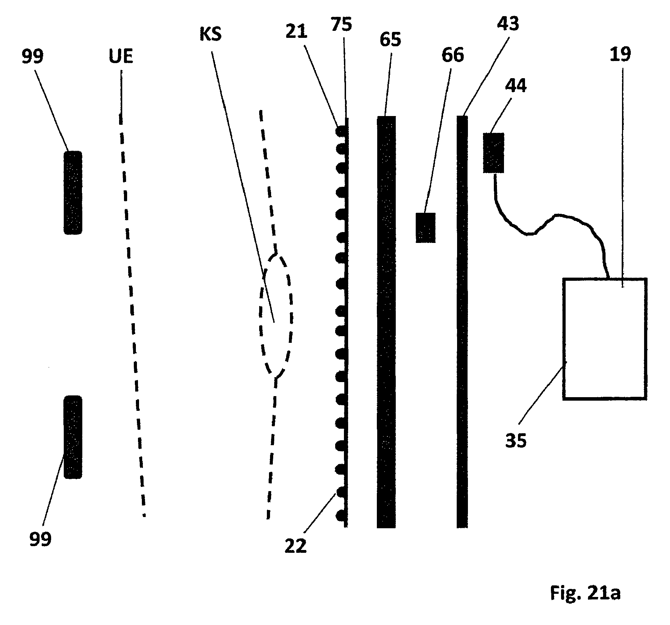

Fig. 21a zeigt eine erfindungsgemäße Vorrichtung mit einer Elektrodenanordnung, einem als Pelotte ausgestalteten Druckkörper 65 und einem Tragelement 43, das als Bandage ausgestaltet ist, in einer "Explosionsdarstellung" für eine Anwendung an einer unteren Extremität UE mit Kniescheibe KS. Die Elektrodenanordnung mit dem Elektrodenfeld kann in Anlage an die Haut platziert werden. Die Vorrichtung weist ferner mindestens eine (Gegen-)Elektrode 99 als Anode zu den als Kathoden ausgestalteten Elektroden 21, 22 der Elektrodenanordnung auf, die hinten am Knie in Anlage auf die Haut gelangen können. Der Vibrationsmotor 66 ist oberhalb der Kniescheibe KS in der Ausnehmung des Druckkörpers 65 bzw. der Pelotte befestigt. Die Pelotte ist zwischen Tragelement 43 (als Textil ausgestaltet) und Elektrodenanordnung angeordnet. Elektrodenanordnung und Tragelement 43 sind unter Ausbildung eines Zwischenraums, in dem sich die Pelotte mit dem Vibrationsmotor befindet, vernäht. Die Elektrodenanordnung und der Vibrationsmotor können über einen oder mehrere nach außen geführte Leiter mittels eines Steckers 44 kontaktiert werden. Die Ansteuerung des Vibrationsmotors 66 und der Elektrodenanordnung erfolgt über eine Steuereinheit 19, 35, die vorzugsweise an dem Tragelement 43 befestigt ist. Vorzugsweise ist die Steuereinheit 19, 35 klein und weist wenig Masse auf. Vorzugsweise ist die Spannungs- bzw. Stromversorgung für die Steuereinheit 19, 35 auch am Tragelement 43 befestigt. Die Ausgestaltung von Steuereinheit 19, 35 und Spannungs- bzw. Stromversorgung ist vorzugsweise derart, dass eine Mobilität des Menschen bzw. Tieres erhalten bleibt.

Eine erfindungsgemäße Vorrichtung kann ein Tragelement umfassen, das den Körper eines Menschen mit dem Bereich der Haut, die die Elektrodenanordnung kontaktieren soll, zumindest teilweise umgibt. Das Tragelement kann ein Textil, eine Folie, ein Kunststoff, Leder oder ähnliches sein. Das Tragelement kann als Band oder als Strumpf oder schlauchförmig ausgestaltet sein. Das Textil kann ein Gestrick sein, das anatomisch formgestrickt ist. Das Tragelement ist vorzugsweise dehnbar und/oder elastisch.

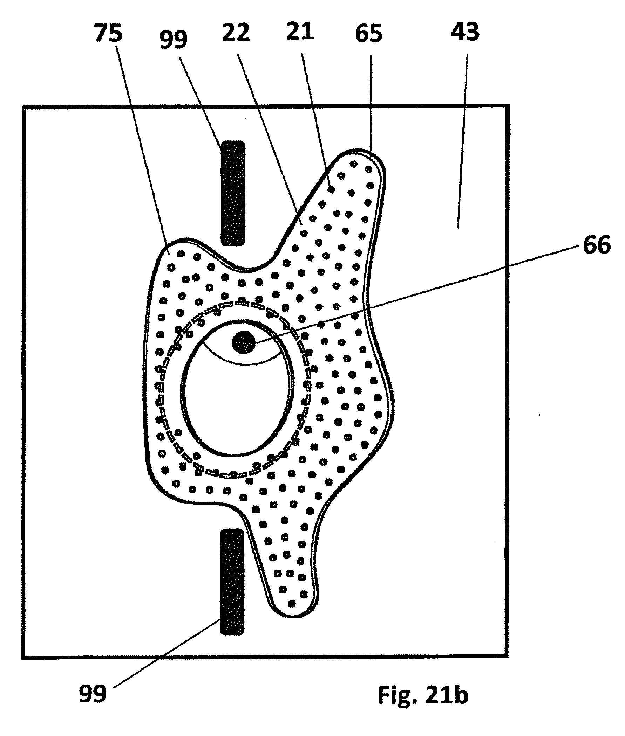

Fig. 21b zeigt eine schematische Vorderansicht aus Blickrichtung des Knies auf die in Fig. 21 a gezeigte Vorrichtung in teilweise geschnittener Darstellung.

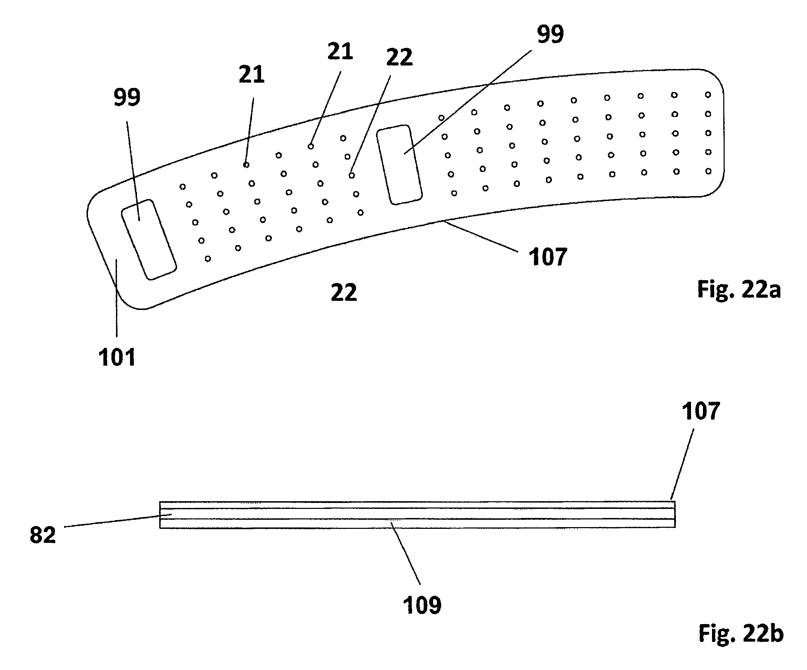

Fig. 22a zeigt schematisch eine bandförmige Vorrichtung, mit der insbesondere ein Phantomschmerz an einem Stumpf einer äußeren Extremität verringerbar sein kann. Auf einem bandförmigen Element 107, das insbesondere als Textil 107 ausgestaltet sein kann, ist eine Elektrodenanordnung aus Elektrodenfeld mit Elektroden 21, 22 und beabstandeter, weiterer Elektrode 99 angeordnet. Neben den punktuell ausgestalteten, insbesondere als Kathoden beaufschlagbaren, Elektroden 21, 22 ist mindestens eine - vorzugsweise zwei - weitere beabstandete Elektrode 99 ausgestaltet. Die weitere beabstandete Elektrode ist vorzugsweise flächig ausgestaltet. Die Vorrichtung umfasst weiter einen als Abstandsgewirke ausgestalteten Druckkörper 82, der zwischen der Elektrodenanordnung mit bandförmigen Element 107 und einem aus Textil oder Gewirk gefertigten Tragelement 109, das als Trägerband ausgestaltet ist, angeordnet ist. Mit zwei Anoden ist eine zweikanalige Ansteuerung der Elektrodenanordnung möglich. Die bandförmige Vorrichtung kann über einen Verschluss 101, der als Klettverschluss oder Steckverschluss ausgestaltet sein kann, in Anlage an die Haut gebracht werden.

Fig. 22b zeigt schematisch die bandförmige Vorrichtung von 22a in einer Ansicht von oben.

Im Nachfolgenden werden drei Untersuchungsstudien, die die Wirksamkeit von erfindungsgemäßen Ausführungsformen zeigen, in Form von Beispielen wiedergegeben:

### Erstes Beispiel:

Untersuchung des Effektes der erfindungsgemäßen Schmerzsuppression auf verschiedene Schmerzmodalitäten mittels Quantitativ Sensorischer Testung (QST) Die Studie zeigt, dass der Einsatz erfindungsgemäßer Elektroden eine Erhöhung der Schmerzschwelle bei Schmerz bewirkt. Eine Langzeithemmung (LTD) der synaptischen Übertragung durch niederfrequente elektrische Stimulation kann (wahrscheinlich) hervorgerufen werden.

### Methode:

Auf Grundlage eines Protokolls zur Quantitativen Sensorischen Testung (QST, Rolke R, Magerl W, Campbell KA, et al., 2006, Quantitative sensory testing: a comprehensive protocol of clinical trials. European Journal of Pain, Vol. 10 (1), S. 77-88; Rolke R, Baron R, Maier C, et al., 2006, Quantitative sensory testing in the German research network of neuropatic pain (DFNS): Standardized protocol an reference values. PAIN, Vol. 123 (3), S. 231-243; Backonja MM, Walk D, Edwards RR, Sehgal N, Moeller-Bertram T, Wasan A, Irving G, Argoff C, Wallace M, 2009, Quantitative sensory testing in measurement of neuropatic pain phenomena and other sensory abnormalities. Clinical Pain, Vol. 25, S. 641-647; Rolke R, 2009, Diagnostischer "Work-up" neuropatischer Schmerzen in der klinischen Praxis: Quantitative sensorische Testung als komplementäres Verfahren zur konventionellen Elektrophysiologie. Klinische Neurophysiologie, Vol. 40, S. 177-182) wird der Einfluss von zwei verschiedenen erfindungsgemäßen Elektrodenanordnungen getestet. Ziel der Testung ist die standardisierte Untersuchung von je zwei symmetrischen Körperarealen. Am Probanden wird stets das stimulierte Hautareal vor der Kontrollseite untersucht. Die Durchführung des gesamten Protokolls für zwei Areale dauert in der Regel 90 Minuten.

### Ablauf und Aufbau:

Die lokale Stimulation erfolgt mit erfindungsgemäßen Elektrodenanordnungen (s.u.) auf dem Unterarm, die mit einem Reizstromgenerator verbunden werden. Testareal rechter oder linker Unterarm mit erfindungsgemäßer Elektrodenanordnung: Kreiselektrode mit Frequenz 1 Hz bei 5min Stimulationsdauer oder Igel-Elektrode mit Frequenz 4Hz bei 15min Stimulationsdauer; Intensität bis 2,5mA. Die Stimulation erfolgt randomisiert entweder als Verum oder Placebo mit einem ineffektiven Stimulationsmuster über 5min (Konzentrische Elektrode) und 15 min (Igelelektrode).

### Aufbau der Elektroden:

1. Konzentrische Elektrode: Große externe Ring-Anode mit einem inneren Durchmesser von 8mm und einem äußeren Durchmesser von 24mm. Schmale Zentrale-Kathode mit einem Durchmesser von 1 mm.
2. Igelelektrode (Matrix Array): Bei der Igelelektrode handelt es sich um ein Multielektroden-Array 4x4cm². Mit Ball-Grid Beschichtung. Anode und Kathode werden wechselseitig geschaltet. Dadurch ergibt sich eine optimale Stimulation der Aö- und C-Nervenfasern die in der Cutis und Subcutis verlaufen. Zur Stimulation der Aß-Fasern wurde eine Vibrationseinheit der Elektrode aufgesetzt.

Folgende Testverfahren haben sich aus der durchgeführten Quantitativ Sensorischen Testung herauskristallisiert und wurden durch mehrere Einzeltests genauer untersucht.

### Mechanische Schmerzschwelle (MPT = mechanical pain threshold)

Dieser Test wurde mit Nadelreizstimulatoren (Pinprick) durchgeführt. Die Schwellenbestimmung erfolgt in fünf Serien auf- und absteigender Stimulusintensitäten. Als Schwelle wurde der geometrische Mittelwert der jeweils fünf gerade über- und unterschwelligen Reizstärken angegeben (modifizierte Grenzwertmethode).

### Druckschmerzschwelle (PPT = pressure pain threshold)

### Mit Hilfe eines Druckalgometers (Kontaktfläche 1cm²) wurde die Schwelle für Druckschmerz über einem Muskel in drei Serien langsam aufsteigender Stimulusintensitäten (0,5kg/s, entspricht ca. 50kPa/s) gemessen. Als Schwelle wird der arithmetische Mittelwert dieser drei Serien angegeben (in kPa).

### Ergebnisse:

### Mechanische Schmerzschwelle (MPT = mechanical pain threshold)

Für N = 39 (n = 2340) wurde bei 1, 3 und 4Hz die mechanische Schmerzschwelle errechnet. Die Ergebnisse verdeutlichen, dass sich für beide verwendete Elektrodentypen die Schmerzschwelle um den Faktor 2-3 erhöht. Für Kontrollareal sowie Placebo beträgt die mechanische Schmerzschwelle zwischen 40-80mN.

Hingegen steigt diese Schwelle auf durchschnittlich (Mittelwert) 100-200mN. Die folgenden Mittelwerte je Elektrodenart werden erreicht:

| Konzentrische Elektrode | | Igelelektrode |
|---|---|---|
| 1 Hz: | 185mN | 98mN |
| 3 Hz: | 123mN | 210mN |
| 4 Hz: | 149mN | 117mN |

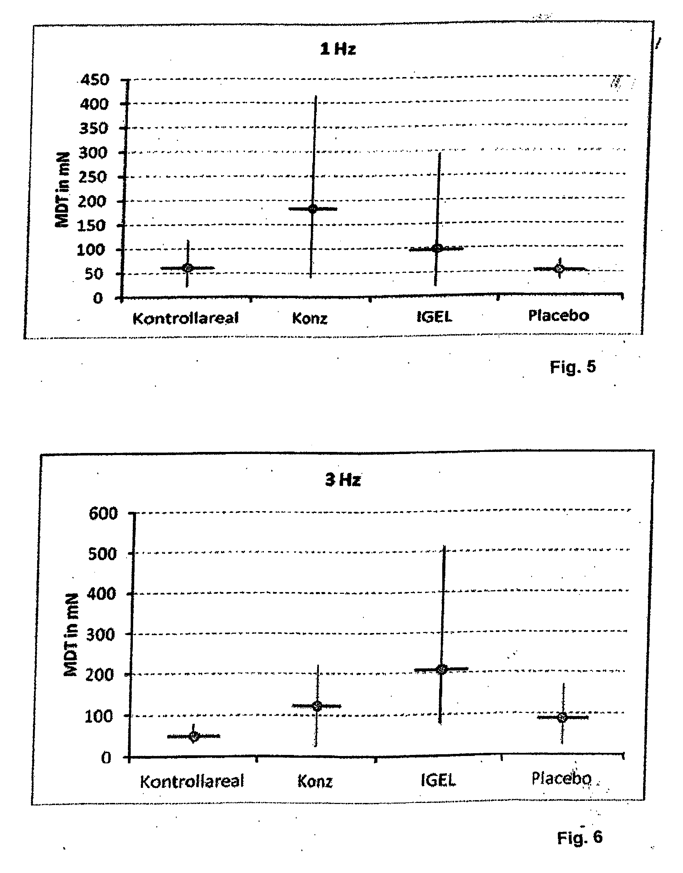

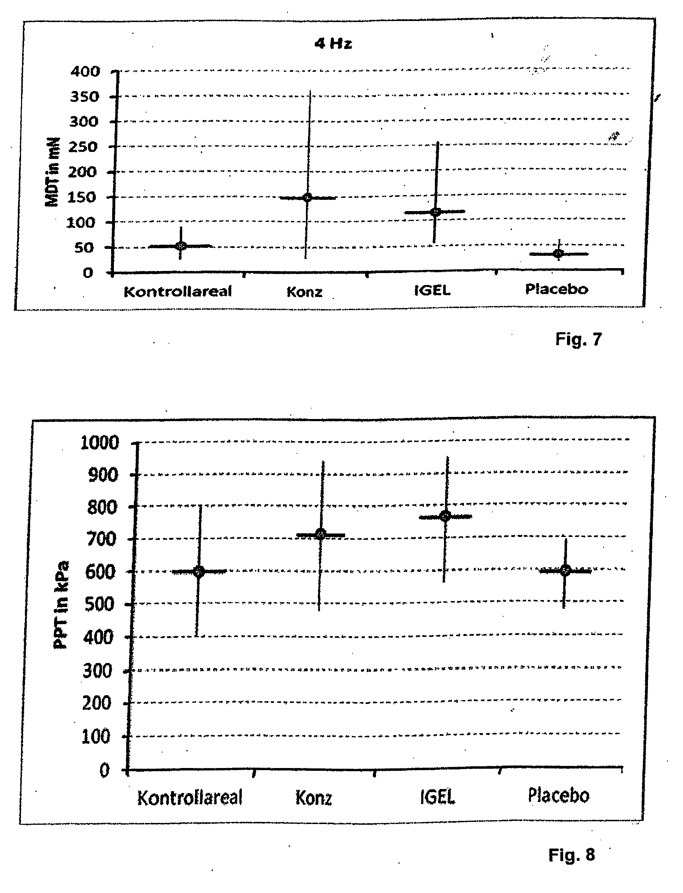

### Figuren 5 bis 8 illustrieren die Ergebnisse.

### Druckschmerzschwelle (PPT = pressure pain threshold)

Für N = 22 (n = 132) wurde die Druckschmerzschwelle im Kontroll- und Testareal nach Stimulation ermittelt. Dabei wurde mit N = 11 für die konzentrische sowie N = 11 für die Igelelektrode getestet. Es zeigt sich nach Stimulation, dass eine Druckschmerzschwellenverschiebung um 23,8% (konzentrische Elektrode) bzw. 18,4% (Igelelektrode) erreicht wird. Figur 16 verdeutlicht die Ergebnisse.

### Zusammenfassung:

Die Studie zeigt, dass eine erfindungsgemäße Elektrodenanordnung signifikanten Einfluss auf die Mechanische Schmerzschwelle und die Druckschmerzschwelle haben.

### Zweites Beispiel:

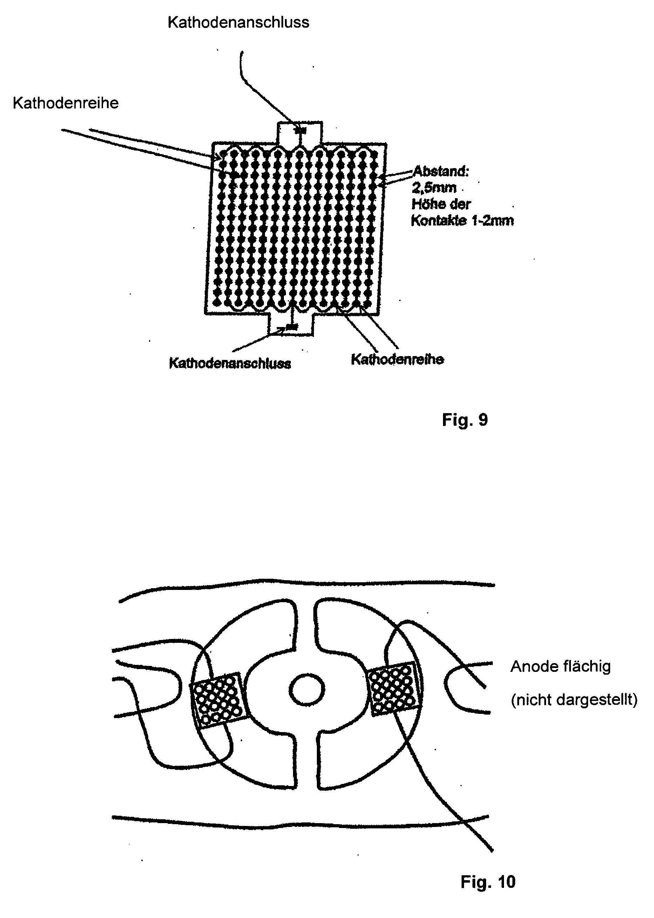

In einer zweiten Versuchsreihe wurde das erarbeitete Funktionsmuster (Bandage mit Igelelektrode) (siehe Figuren 9 und 10) bei Druckschmerz am Knie getestet.

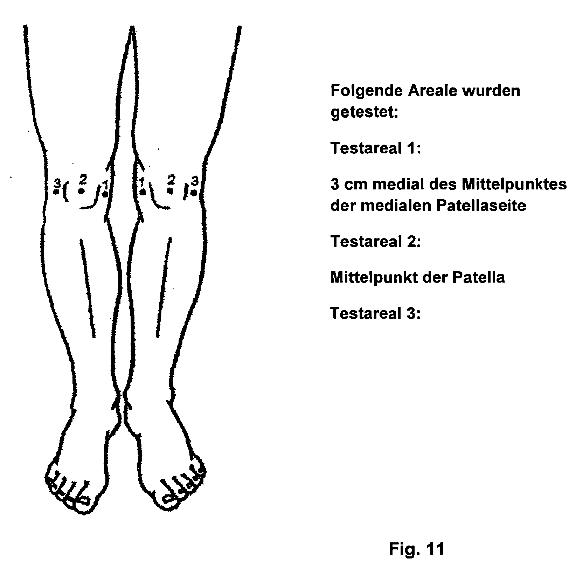

Zur Testdurchführung wurde ein Handalgometer (Somedic AB, Sweden) genutzt. Die Probenspitze (1cm²) wurde an drei vorgegeben Haut-Punkten der peripatellaren Region getestet (siehe Figur 11). Der Druck wurde mit 30kPa/s aufgebracht, bis der Proband den Druck als schmerzhaft angab.

Es wurden sechs Personen mit folgendem Ablaufmuster getestet:
- Jede Person wurde insgesamt 6 Tage ä 3 Durchgänge am Universitätsklinikum Bonn getestet (6*3=18 Durchgänge).
- Tag 1 und 2 wurden ohne Stimulation durchgeführt (BASELINE).
- Tag 3 und 4 wurden mit Sham durchgeführt (SHAM).
- Tag 5 und 6 wurden mit dem Funktionsmuster (inkl. Elektrodenanordnung) durchgeführt.
- Jede Stimulation dauerte 15 Minute (effektive Stimulationsmuster siehe erstes Beispiel) und wurde nach 45 Minuten wiederholt.
- Die Druckschmerzschwelle (PPT) wurde in folgenden Intervallen getestet: 0, 5, 15 und 30 Minuten.

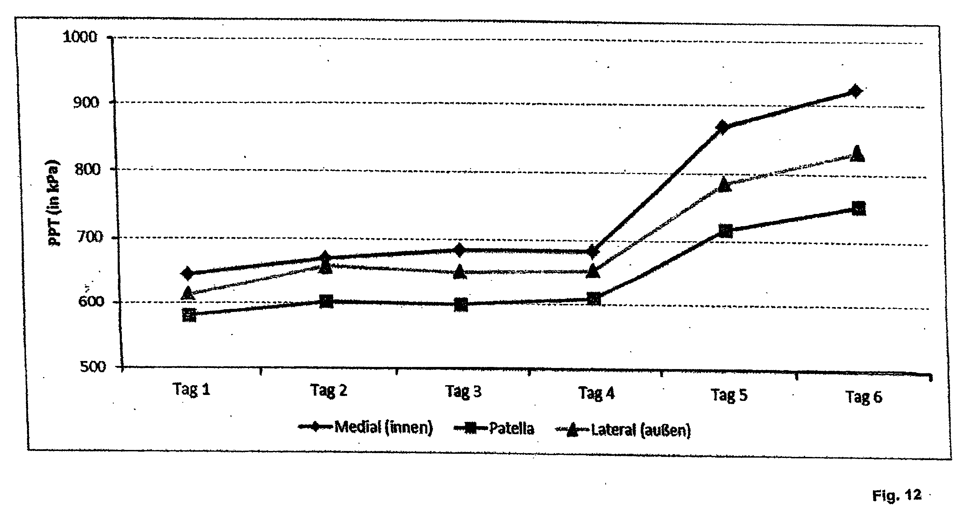

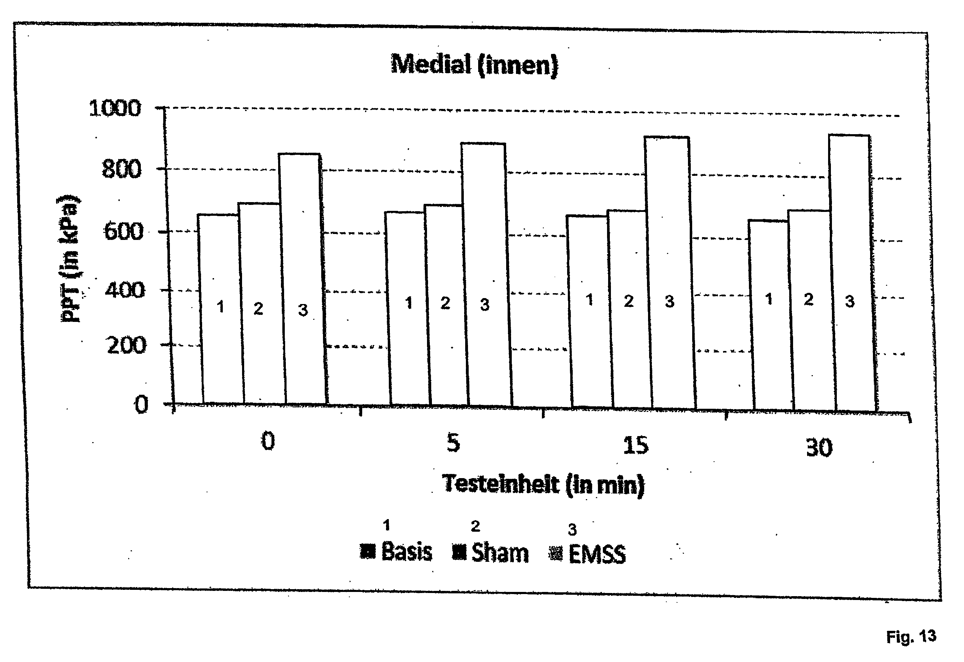

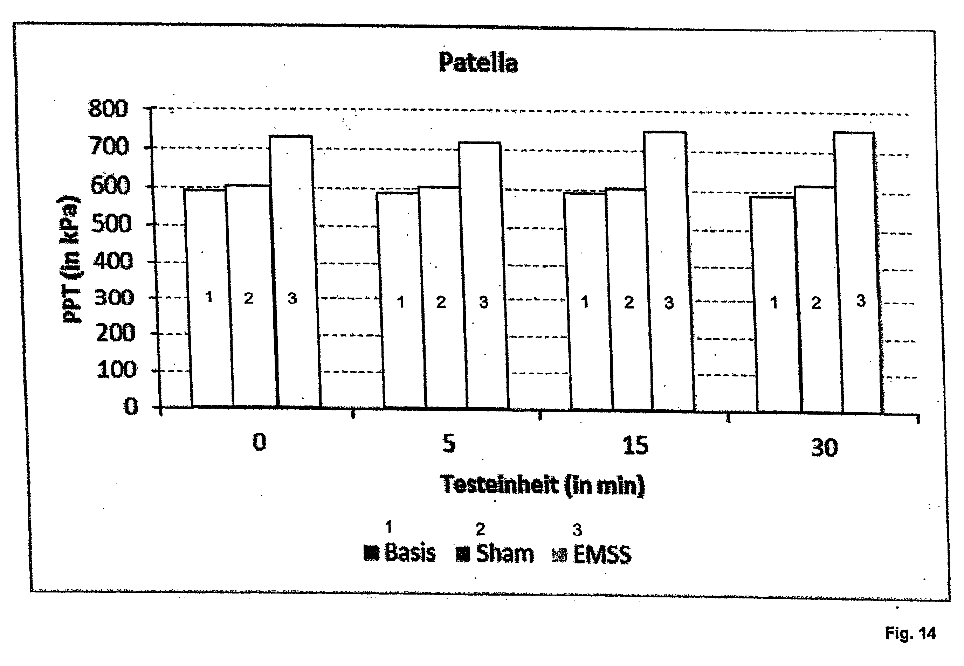

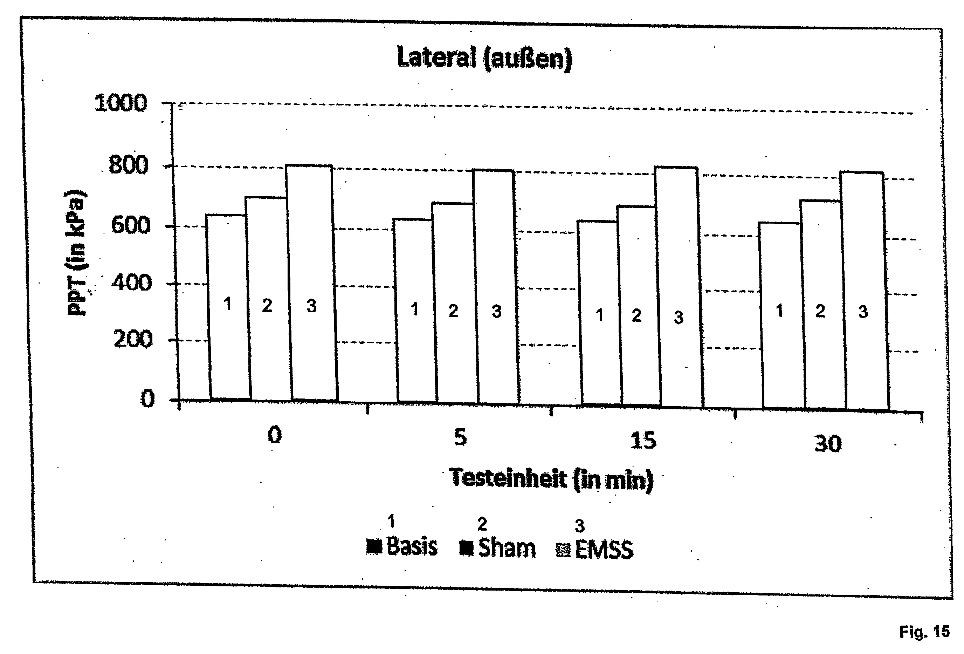

Die in den Fig. 12 bis 15 dargestellten Graphen fassen die ersten gesammelten Daten zusammen:

Folgende Testergebnisse können zusammengefasst werden:
- Im Testareal 2 (Patella) wird am schnellsten die Druckschmerzschwelle erreicht (bei ca. 700kPa) im Vergleich zur lateralen (ca. 800kPa) und medialen (ca. 850kPa) Seite.
- Nach Stimulation wird die Schmerzschwelle deutlich erhöht, sowohl im Vergleich zu BASELINE als auch zu SHAM.
- Das Maximum der Schmerzschwelle wird erreicht im Intervall 5-10 Minuten nach Stimulation. Die erhöhte Schmerzschwelle bleibt auch noch bis 30 Minuten nach Stimulation bestehen.
- Bei einer wiederholten Stimulation (45 Minuten Takt) erhöht sich die Schmerzschwelle. An Tag 2 beginnt diese zwar wieder erniedrigt, aber bereits erhöht im Vergleich zu Tag 5.

Es zeigt sich, dass das Funktionsmuster mit der benutzten EMSS-Elektrode eine nachweisbare schmerzreduzierende Wirkung erzielt.

### Drittes Beispiel:

Die Elektrodenanordnung ist mit der Elektrodenanordnung der zweiten Versuchsreihe identisch (flächige Elektrodenanordnung in einem Array). Zusätzlich wurde ein mit der Elektrodenanordnung verbundener, die Elektrodenanordnung in Schwingungen versetzender Vibrationsmotor verwendet. Als Vibrationsmotor wurde ein PicoVibe™-Motor 4mm Vibration Motor, 8mm Type gemäß Datenblatt 304-008 von PRECISION MICRODRIVES™ verwendet.

Die Versuchsreihe mit 5 Probanden zeigte bei einer Stimulationsdauer von 5min eine gegenüber der ersten Versuchsreihe weiter erhöhte Schmerzschwelle. Die zusätzliche Vibration führte damit zu einer weiteren Schmerzreduktion bzw. Schmerzunterdrückung.

In einer nachfolgend dargestellten Studie wurde die Matrixanordnung mit Gegenelektrode verglichen mit einer konzentrischen Elektrodenanordnung. Der Vergleich der Matrixanordnung mit Gegenelektrode und konzentrischer Elektrodenanordnung zeigte nach einer konditionierenden Stimulation mit 4Hz ein divergentes Muster. Dem Trend nach beeinflusste die konzentrische Elektrode mehr die thermischen Wahrnehmungs- und Schmerzschwellen, während die Reizung mittels Matrixelektrode zu einer Erhöhung mechanischer Schwellen führte. Diese Effekte waren nach 4Hz-Testreizung stärker ausgeprägt als unter einer Kontrollbedingung mit 30Hz, die nur für einzelne Parameter wie z.B. den Druckschmerz eine optimale Shamfrequenz darstellt. Beachtenswert ist, dass nach einer 4Hz-Stimulation mit der Matrixelektrode und Gegenelektrode auch die Tiefenschmerzempfindlichkeit reduziert war.

Nach niederfrequenter elektrischer Stimulation (LFS) zeigten gesunde Probanden im Rahmen der Studie im Sinn einer Feasibility-Studie hoch signifikante Veränderungen des somatosensorischen Phänotyps. Nach LFS fand sich ein differenziertes Muster sensibler Minuszeichen - je nach Elektrodenart und Reizfrequenz.

### Thermische und mechanische Wahrnehmungsschwelle

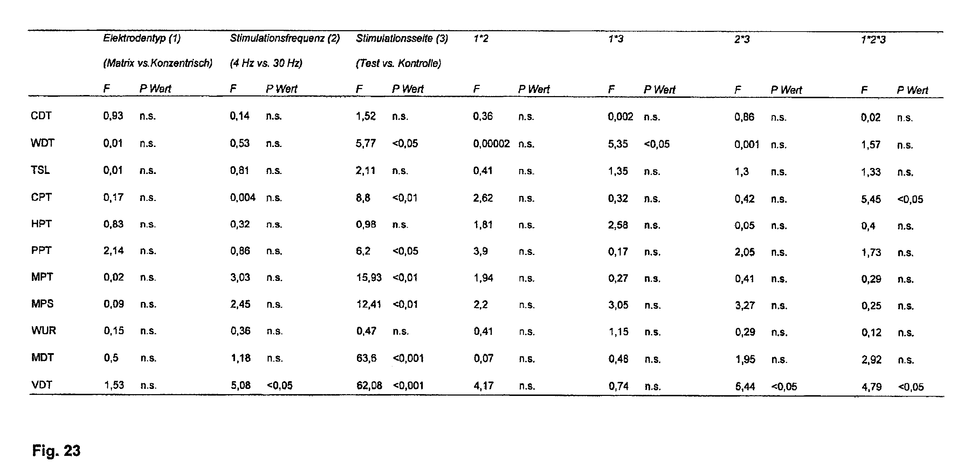

Nach LFS kam es zu einem Funktionsverlust im Bereich der mechanischen deutlich mehr als im Bereich thermischen Wahrnehmungsschwellen. Die mechanische Hypästhesie (VDT, MDT) war dabei sehr viel deutlicher als die thermische Hypästhesie (TSL, WDT, CDT) ausgeprägt (tabellarisch dargestellte Varianzanalyse ANOVA und QST-Rohwerte in Fig. 23 und Figuren 24 und 25).

Fig. 23 ist eine Darstellung der Varianzanalyse (3-faktorielle ANOVA für Messwiederholungen). Die ANOVA zeigt für die meisten mechanischen mehr als für thermische QST-Parameter Stimulationseffekte über dem Testort nach Konditionierung. Die Haupteffekte waren zwischen den Elektrodentypen und Stimulationsfrequenzen grundsätzlich nicht signifikant verschieden, abgesehen von einem signifikanten Effekt der Stimulationsfrequenz (4Hz vs. 30Hz) auf die Vibrationsschwelle. Hier zeigte sich auch eine signifikante 2-fach- und 3-fach-Interaktion der Testfaktoren als Hinweis darauf, dass die Vibrationsschwelle über dem Testort nach Konditionierung mit der Matrixelektrode bzw. der Elektrodenanordnung mit Elektrodenfeld und weitere beabstandeter Elektrode bei 4Hz-Reizung am deutlichsten verändert war.

### Thermische und mechanische Schmerzschwelle

LFS zeigte nur für Kältereize (CPT; konzentrische Elektrode) eine Kältehypoalgesie (p<0,01). Die Druckschmerzschwelle (PPT; p<0,05) war ebenso wie die mechanische Schmerzschwelle für Nadelreize (MPT; p<0,01; MPS; p<0,001) vornehmlich nach Stimulation mit einer Matrixelektrode erhöht (mechanische Hypoalgesie). Eine dynamische mechanische Allodynie oder ein veränderter Wind-up Quotient konnten nicht nachgewiesen werden.

### Varianzanalyse (ANOVA in Fig. 23)

### Einfluss des Elektrodentyps (Faktor 1) auf die Veränderung der QST-Parameter

Die ANOVA zeigt für den Faktor Elektrodentyp für keinen der QST-Parameter einen signifikanten Haupteffekt. Allein die Warmschwelle zeigte eine signifikante Interaktion mit dem Faktor 3 "Stimulationsseite" (kontralaterale Baseline-Bedingung vs. Testreiz). Hier war die Warmschwelle bei Stimulation mit 30Hz-Testreiz nur bei Reizung mit der konzentrischen Elektrode erhöht. Dieser Effekt ist durch einzelne Ausreißer bedingt und sollte nicht überbewertet werden.

### Effekt der Stimulationsfrequenz (Faktor 2)

Nur für den QST-Parameter VDT fand sich ein signifikanter Haupteffekt (F=5,08; p<0,05). Die Vibrationsschwelle war sowohl nach Stimulation mit einer Matrix- wie auch einer konzentrischen Elektrode erhöht. Dieser Effekt war bei den Testfrequenzen 4Hz und 30Hz für die beiden Elektroden jeweils unterschiedlich stark ausgeprägt, mit den deutlicheren 4Hz-Effekten für die Matrixelektrode, bei deutlicherem 30Hz-Effekt nach Reizung mit der konzentrischen Elektrode. Dies erklärt die entsprechenden 2-fach- (Faktoren 2x3) und 3-fach-Interaktionen (Faktoren 1x2x3).

### Einfluss der Stimulationsseite (Faktor 3: Baseline-Bedingung vs. Testreiz)

Bis auf den Wind-up Quotienten zeigen mit Betonung für die Wahrnehmungsschwellen alle mechanischen QST-Parameter signifikante Haupteffekte, die auf eine signifikante Veränderung der mechanischen Sensitivität durch die konditionierende 4Hz- bzw. 30Hz-Stimulation hinweisen. Am deutlichsten ausgeprägt war eine mechanische Hypästhesie (MDT; F=63,6; VDT; F=62,1; alle p<0,001). Dann folgte eine mechanische Hypoalgesie für Nadelreize (MPT; F=15,9; MPS; F=12,4; alle p<0,01). Nur nach Reizung mit der Matrixelektrode bei 4Hz fand sich auch eine Druckhypoalgesie (PPT; F=6,2; p<0,05). Die thermischen QST-Parameter waren weniger deutlich verändert im Sinn einer Kältehypoalgesie (CPT; F=8,8; p<0,01) und Wärmehypästhesie (WDT; F=5,8; p<0,05).

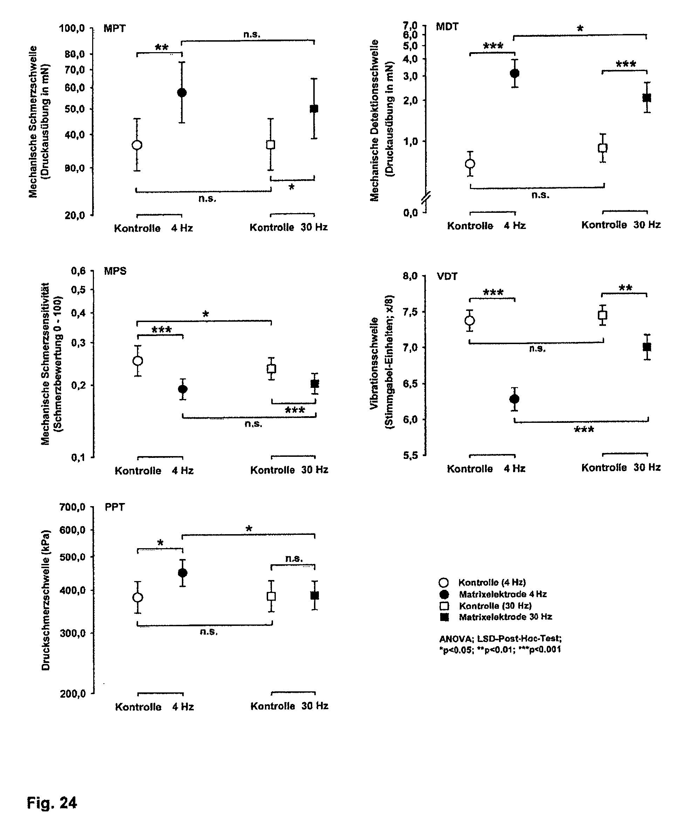

Fig. 24 zeigt eine Übersicht der QST-Rohdaten. Nach Konditionierung mit einer Matrixelektrode waren alle mechanischen Wahrnehmungs- und Schmerzschwellen (Ausnahme Wind-up Quotient) nach Reizung mit 4Hz viel deutlicher als nach Reizung mit 30Hz gegenüber der Baseline-Bedingungen erhöht. Nur für die Druckschmerzschwelle (PPT) als Parameter einer veränderten Tiefenschmerzempfindlichkeit entsprach die Stimulation mit 30Hz einer optimalen Shambedingung.

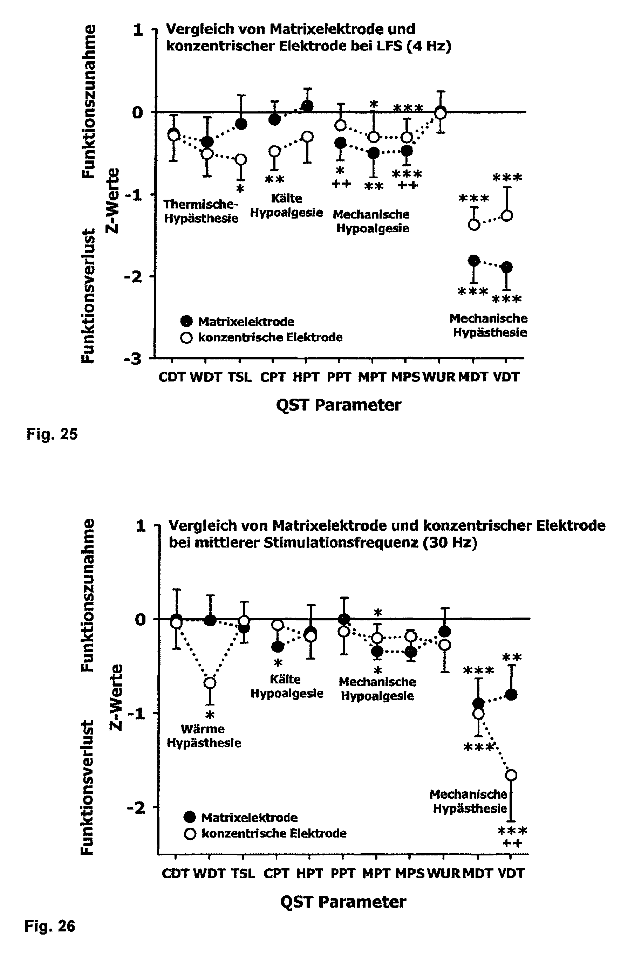

Die Fig. 25 zeigt einen Vergleich zwischen Matrixelektrode (Elektrodenfeld mit beabstandeter Elektrode) und konzentrischer Elektrode nach 4Hz Stimulation. Dargestellt sind Z-Werte auf der Basis von QST-Log-/Rohdaten nach der Formel Z=(MWBaseline-MWStimulation)/SDBaseline. Ein Z-Wert von "0" entspricht dem MW entsprechend der unkonditionierten Kontrollbedingung. Positive Z-Werte weisen auf eine Funktionszunahme, negative auf einen Funktionsverlust im sensorischen System hin.

Fig. 25 zeigt ein vollständiges QST-Profil (Z-Werte) nach niederfrequenter Stimulation (LFS; 4Hz). Dargestellt sind die Z-Profile nach konditionierender Reizung mit einer Matrix- sowie einer konzentrischen Elektrode. Die Matrix- sowie die konzentrische Elektrode zeigten bei 4Hz-Stimulation ein divergentes Muster sensorischer Veränderungen. Nach Reizung mit der konzentrischen Elektrode waren die thermischen Wahrnehmungs- und Schmerzschwellen dem Trend nach höher, bei Reizung mit der Matrixelektrode alle mechanischen Schwellen. Dieser Effekt war für den Vergleich der Elektroden aber nur für PPT (Druckschmerzschwelle) und MPS (mechanische Schmerzsensitivität einschließlich überschwelliger, leichter Schmerzreize) signifikant (ANOVA, LSD post hoc-Test; p<0,01).

### Elektrodenvergleich nach 30Hz Stimulation

Fig. 26 zeigt einen Vergleich zwischen Matrixelektrode (Elektrodenfeld mit beabstandeter Elektrode) und konzentrischer Elektrode nach 30Hz Stimulation. Dargestellt sind Z-Werte auch hier entspricht "0" dem MW entsprechend der unkonditionierten Kontrollbedingung. Positive Z-Werte weisen auf eine Funktionszunahme, negative auf einen Funktionsverlust im sensorischen System hin. Nach 30Hz Konditionierung zeigen sich keine Effekte auf den Tiefenschmerz.

Die Fig. 26 zeigt, sowohl für die Matrix- als auch für die konzentrische Elektrode, ein vollständiges QST-Profil nach Stimulation mit der angenommenen Shamfrequenz (30Hz). Für die thermischen Wahrnehmungsschwellen bestanden für beide Elektroden nahezu Baselinebedingungen. Nach der Stimulation mit der konzentrischen Elektrode zeigte sich, bedingt durch Ausreißer, eine Wärme-Hypästhesie (WDT; p<0,05). Für die Matrixelektrode bestand eine leichte Kälte-Hypoalgesie (CPT; p<0,05). Nach Nadelreizen konnte für beide Elektroden eine mechanische Hypoalgesie (MPT; p<0,05) erreicht werden. Für die Druckschmerzschwelle (PPT) trat anders als nach 4 Hz-Stimulation keine Schwellenwertverschiebung auf. Die mechanische Detektions-(MDT, beide Elektrodenarten p<0,001) und die Vibrationsschwelle (VDT; Matrixelektrode p<0,01; konzentrische Elektrode, p<0,001) konnten eine hochsignifikante mechanische Hypästhesie aufweisen.

### Vergleich der Stimulationsfrequenzen 4Hz vs. 30Hz

Beim Vergleich der Stimulationsfrequenzen untereinander (Fig. 25 und 26) konnten keine signifikanten ANOVA-Haupteffekte gefunden werden. Nur für VDT konnte ein signifikanter Effekt (p<0,05) bei einer Stimulationsfrequenz von 30Hz zwischen den verschiedenen Elektroden beobachtet werden. Ebenso wie schon in Vorversuchen der Anmelder, fanden sich bei 30Hz-Stimulation mit einer Matrixelektrode keine Unterschiede zwischen der Druckschmerzschwelle nach dieser Konditionierung im Vergleich mit der nicht konditionierten Kontrollbedingung (Baseline-Messung am kontralateralen Unterarm).

### Faktorenanalyse des somatosensorischen Phänotyps nach 4Hz-Konditionierung mit Matrixelektrode

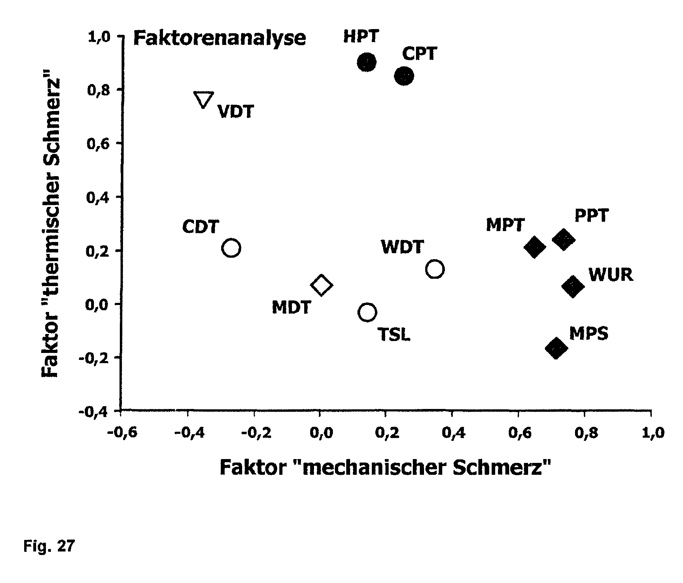

Die beste Varianzaufklärung (vgl. folgende Tabelle "Varianzaufklärung") gelang in einem 4-faktoriellen Modell (78,5% aufgeklärte Gesamtvarianz) mit den Faktoren thermischer Schmerz, mechanischer Schmerz, thermische Wahrnehmung, mechanische Wahrnehmung mit der Ausnahme VDT, die signifikant negativ auf den Faktor thermischer Schmerz lud (in Fig. 27 dargestellt als Absolutwert).

**Tabelle "Varianzaufklärung"**

| x-faktorielles Modell | Varianzaufklärung |
|---|---|
| 2-faktorielles Modell | 50,0% |
| 3-faktorielles Modell | 67,7% |
| 4-faktorielles Modell | 78,5% |

Die ausgewählten Faktoren des Modells wurden mit Faktor 1 thermische Detektionsschwellen, Faktor 2 mechanischer Schmerz, Faktor 3 thermischer Schmerz und Faktor 4 mechanische Detektionsschwellen beschrieben (Tabelle "Faktorladungen").

**Tabelle "Faktorladungen" (Markierte Ladungen >0,6)**

| | Faktor 1 | Faktor 2 | Faktor 3 | Faktor 4 |
|---|---|---|---|---|
| CDT | 0,87 | -0,27 | 0,21 | -0,08 |
| WDT | 0,79 | 0,35 | 0,13 | 0,26 |
| TSL | 0,94 | 0,14 | -0,03 | 0,11 |
| CPT | 0,13 | 0,25 | 0,85 | -0,18 |
| HPT | -0,03 | 0,14 | 0,90 | 0,32 |
| MDT | -0,13 | 0,00 | -0,07 | -0,92 |
| MPT | -0,16 | 0,65 | 0,21 | -0,32 |
| MPS | 0,33 | 0,71 | -0,16 | -0,04 |
| WUR | 0,00 | 0,76 | 0,07 | -0,03 |
| VDT | -0,24 | 0,36 | -0,77 | 0,05 |
| PPT | 0,11 | 0,73 | 0,24 | 0,36 |

Fig. 27 zeigt eine Faktorenanalyse. Die Darstellung zeigt die Gruppierung einzelner QST-Parameter nach dem Ladungsverhalten auf die Faktoren "thermischer Schmerz" und "mechanischer Schmerz" nach vorausgehender konditionierender Stimulation mit einer Matrixelektrode [Elektrodenfeld mit beabstandeter Elektrode] (4Hz) - signifikante Ladungen dargestellt mit geschlossenen Symbolen. PPT als Parameter einer veränderten mechanischen Tiefenschmerzsensitivität zeigt ein gleichartiges signifikantes Ladungsverhalten wie die QST-Parameter MPT, MPS und WUR, die die Sensitivität der Haut gegenüber spitzen mechanischen Reizen (Nadelreiz) repräsentieren. Die Hitze- und Kälteschmerzschwelle zeigt demgegenüber ein signifikant anderes Ladungsverhalten auf den Faktor "thermischer Schmerz". Die thermischen und mechanischen Wahrnehmungsschwellen (CDT, WDT, TSL, MDT) zeigen hier kein eindeutiges Ladungsmuster. Allein die Vibrationsschwelle lädt signifikant negativ auf den Faktor "thermischer Schmerz" - hier dargestellt als Absolutwert.

### Methodik und "Feasibility"

Die Quantitative Sensorische Testung (QST) eignet sich als Verfahren zur experimentellen Schmerzmessung, da sich die Performance peripherer Nervenfasern (Aβ-, Aδ- und C-Fasern) und deren zentrale Projektionswege ins Gehirn untersuchen lassen (Pfau, Geber, Birklein, Treede: Quantitative sensory testing of neuropathic pain patients: potential mechanistic and therapeutic implications; Curr Pain Headache Rep 2012; 16: 199-206). Mit Hilfe der QST lässt sich ein individuelles sensorisches Profil für jeden getesteten Probanden erstellen (Krumova et al., 2012). Trotzdem muss berücksichtigt werden, dass es sich bei diesem Verfahren um ein subjektives standardisiertes algesimetrisches Messverfahren handelt, bei dem mit kalibrierten Reizen unterschiedlicher sensorischer Modalität gearbeitet wird (Krumova, Geber, Westermann, Maier. Neuropathic Pain: is Quantitative Sensory Testing Helpful? Current Diabetes Reports 2012; 12: 393-402; Rosenow, Rosenow-Tronnier-Göbel, Tronnier. Neurogener Schmerz: Management von Diagnostik und Therapie. Heidelberg: Springer, 2005). Diese Art der Testung gehört zu den psychophysischen Messmethoden, bei denen der Zusammenhang zwischen physikalischen Eigenschaften eines Reizes und der subjektiven Empfindung bewertet wird (Zaslansky, Yarnitsky. Clinical applications of quantitative sensory testing (QST). J Neurol Sci 1998; 153: 215-238). Die Auswertbarkeit und die Qualität der Befunde sowie die Wiederholbarkeit und Vergleichbarkeit ist abhängig vom Verständnis und der Motivation des Patienten bzw. Probanden (Rosenow, Rosenow-Tronnier-Göbel, Tronnier. Neurogener Schmerz: Management von Diagnostik und Therapie. Heidelberg: Springer, 2005). Um eine optimale Reliabilität zu gewährleisten und systematische Fehler zu vermeiden, müssen die Handlungsanweisung und die Messung standardisiert mit Hilfe kalibrierter Reize durchgeführt werden (Krumova, Geber, Westermann, Maier. Neuropathic Pain: is Quantitative Sensory Testing Helpful? Current Diabetes Reports 2012; 12: 393-402).

Bei der statistischen Vergleichbarkeit von QST-Normwerten spielen bei der Erhebung der Daten Einflussgrößen wie beispielsweise Methode, Untersuchungsareale, Vorerkrankungen, Modalität, das Alter und Geschlecht eines Probanden eine Rolle (Meh, Denislic. Quantitative assessment of thermal and pain sensitivity. J Neurol Sci 1994; 127: 164-169; Wagner. Klinisch-experimentelle Untersuchung mittels Quantitativ Sensorischer Testung (QST) bei ischämisch bedingten Funktionsdefiziten der unteren Extremität an Patienten mit peripherer arterieller Verschlusskrankheit (pAVK); Yarnitsky, Sprecher. Thermal testing: normative data and repeatability for various test algorithms. J Neurol Sci 1994; 125: 39-45). Die Testung wurde in der Studie bei einem gesunden Probandenkollektiv durchgeführt. Außerdem bestanden bei den Probanden keine Vorerfahrungen mit Elektrostimulation. Ein Bias durch spezifische Erwartungen der Probanden war nicht anzunehmen. Somit waren die Voraussetzungen für eine reproduzierbare Messung erfüllt. Passend hierzu konnten bei allen Probanden alle Messwerte vollständig für alle QST Parameter, Elektrodentypen und Testfrequenzen erhoben werden.

Die Ergebnisse der QST nach Konditionierung mit verschiedenen Elektrodenarten zeigen auch bei gesunden Probanden eine Modulation sensorischer Schwellen.

### Beeinflussbarkeit des somatosensorischen Phänotyps

Über die Erfassung aller zuvor beschriebenen QST-Parameter lässt sich der vollständige somatosensorische Phänotyp als Empfindlichkeitsprofil gegenüber den getesteten Reizparametern abbilden. Nach konditionierender, niederfrequenter Stimulation (LFS = low frequency stimulation) mit 4Hz zeigte sich bei den untersuchten Probanden eine Abnahme der Schmerzempfindlichkeit und auch eine verringerte Sensitivität gegenüber thermischen Wahrnehmungsreizen und leichter Berührung sowie Vibration. Diese Phänomene einer reduzierten Sinneswahrnehmung waren für den Fall einer Abnahme der Tiefenschmerzempfindlichkeit nicht auf den Ort der Stimulation beschränkt. Nach kutaner Neurostimulation wäre hier keine Abnahme der Tiefenschmerzempfindlichkeit zu erwarten gewesen. Mindestens dieses Phänomen weist darauf hin, dass im Rahmen der niederfrequenten kutanen Neuromodulation auch eine zentralnervös (spinal) vermittelte Abnahme der Prozessierung sensorischen Inputs stattfand, der auch als synaptische Langzeitdepression (LTD = long term depression) bezeichnet wird. Für die Reduzierung der mechanischen Schmerzempfindlichkeit war dieser Befund am deutlichsten bei Stimulation mit einer Matrixelektrode ausgeprägt.

### Thermische und mechanische Wahrnehmungsschwellen

Betrachtet man die thermischen Wahrnehmungsschwellen für Kälte und Wärme nach LFS, so zeigt sich eine leichte Schwellenerhöhung für CDT (cold detection threshold) und WDT (warm detection threshold). Diese Tendenz zeigt sich nach Konditionierung für beide Elektrodenarten. Die Ergebnisse deuten darauf hin, dass Aδ- und C-Faser-vermittelte Reize durch LFS weniger effektiv verarbeitet werden - möglicherweise durch eine reduzierte Prozessierung bei der Übertragung von der peripheren Faserendigung im Hinterhorn des Rückenmarks auf das jeweilige erste zentrale sensorische Neuron. Auch die mechanischen Wahrnehmungsschwellen werden durch beide Elektrodenarten beeinflusst. So kommt es nach niederfrequenter Reizung sowohl mit der Matrix- wie auch der konzentrischen Elektrode zu einer deutlich signifikanten mechanischen Hypästhesie für leichte Berührungsreize (MDT) und Vibration (VDT). Offenbar werden auch Aß-Faser-vermittelte Reize weniger gut nach LFS prozessiert. Dieser Befund weist darauf hin, dass niederfrequente Stimulation am ehesten die zentralnervöse Verarbeitung sowohl thermischer wie mechanischer nichtschmerzhafter Reize reduziert. Wahrscheinlich liegt diesem Prozess eine Form der spinalen LTD zugrunde, die die Weiterleitung von Reizen sowohl über den spinothalamischen Trakt (zentrale Projektionsbahn für die Schmerzreize und Temperatur) als auch über das lemniskale System (Berührung, Vibration, leichter Druck) hemmt.

### Thermische und mechanische Schmerzschwellen

Thermische und mechanische Schmerzschwellen verändern sich nach Konditionierung nicht gleichsam. Nur die Stimulation durch eine konzentrische Elektrode löst thermische Schwellenwertänderungen aus und führt für die Kältewahrnehmung zu einer signifikanten Kältehypoalgesie (verminderte Kälteschmerzempfindlichkeit). Im Unterschied hierzu führen beide Elektrodenarten zu einer Erhöhung mechanischer Schmerzschwellen. Während die konzentrische Elektrode hier allein zu einer reduzierten kutanen Sensitivität gegenüber spitzen mechanischen Reizen führt (Nadelreize), kommt es nach niederfrequenter Reizung mit einer Matrixelektrode neben dieser Nadelreizhypoalgesie (= Pinprick-Hypoalgesie) ebenfalls zu einer reduzierten Empfindlichkeit gegenüber stumpfem Druck (Druckhypoalgesie). Dieser Befund ist neu und beachtenswert, da bisher stets von der Annahme ausgegangen worden war, dass niederfrequente elektrische Stimulation kutaner Afferenzen zu einer homotopen spinalen Langzeitdepression führt, deren Effekte auf den Wirkort der Konditionierung beschränkt bleibt. Mit homotopen Effekten ist hier die Beteiligung nur weniger Synapsen an diesem spinalen Lernphänomen gemeint. Tatsächlich zeigte sich in der Studie hier aber auch eine reduzierte Tiefenschmerzempfindlichkeit, die auch nach Hautanästhesie (hier nicht gezeigtes Zusatzexperiment, siehe hierzu auch nächster Abschnitt) zwischen Konditionierung und Druckmessung erhalten blieb. Dieser Befund kann nur als zentral vermitteltes Phänomen erklärt werden, das über die Reizung kutaner Afferenzen sekundär auch zu einer verminderten Druckschmerzempfindlichkeit von Afferenzen tieferer Gewebe wie z.B. Muskulatur geführt hat. Hier kann es sich nicht allein um einen homotopen Vorgang gehandelt haben. Vielmehr muss von einer heterotopen spinalen LTD nach kutaner Neuromodulation ausgegangen werden, die hier erstmals am Menschen nachgewiesen werden konnte.

### Kutane Neuromodulation: Homotope oder auch heterotope Effekte?

Um Effekte der kutanen Neuromodulation auf periphere Nervenfasern in der Haut als Ursache einer erhöhten Druckschmerzschwelle auszuschließen wurde im Nachgang an die hier berichtete Studie in einem nicht verblindeten Zusatz-Versuch an fünf Probanden nachgewiesen, dass die Druckschmerzschwelle (PPT) auch nach einer oberflächlichen Lokalanästhesie (EMLA-Salbe, 5%ig) der Haut keine signifikanten Änderungen gegenüber den zuvor gefundenen Originaldaten der 16 Testprobanden der hier dargestellten, doppel-blinden, randomisierten Cross-over-Studie zeigte. Dieses Zusatzexperiment belegt, dass die reduzierte Tiefenschmerzempfindlichkeit nicht als Epiphänomen einer veränderten Hautsensibilität erklärt werden kann. Ein Erklärungsansatz ist eher in einer zentralen Hemmung des Schmerz verarbeitenden Systems zu suchen. Welcher zentrale Mechanismus durch die LFS angesprochen wird, ist bisher nicht bekannt. So kann die gezeigte Schwellenwertverschiebung ihren Ursprung sowohl auf Höhe des dorsalen Hinterhorns aber auch in höheren sensorischen Zentren, wie beispielsweise Thalamus oder dem somatosensorischem Kortex haben.

Ex-Vivo-Untersuchungen an Rückenmarksquerschnitten zeigten, dass vor allem die oberflächlichen Schichten des Rückenmarks (Lamina I / spinobrachiale Neurone, Abb. 23) eine Rolle bei der Aktivierung kutaner Afferenzen spielen. Für das nozizeptive System konnte nachgewiesen werden, dass es durch LTP (long term potentiation) zu einer sekundären mechanischen Hyperalgesie kommt. LTP kann sowohl homosynaptisch mit einer Hyperalgesie an der Stimulationsstelle oder heterosynaptisch mit einer Hyperalgesie in angrenzenden Hautpartien ausgeprägt sein. Für LTD können ähnliche Prozesse angenommen werden. Eine heterosynaptische LTD am Menschen mit Abnahme der Tiefenschmerzempfindlichkeit nach kutaner Reizung konnte nach unserem Wissen bisher jedoch noch nie gezeigt werden.

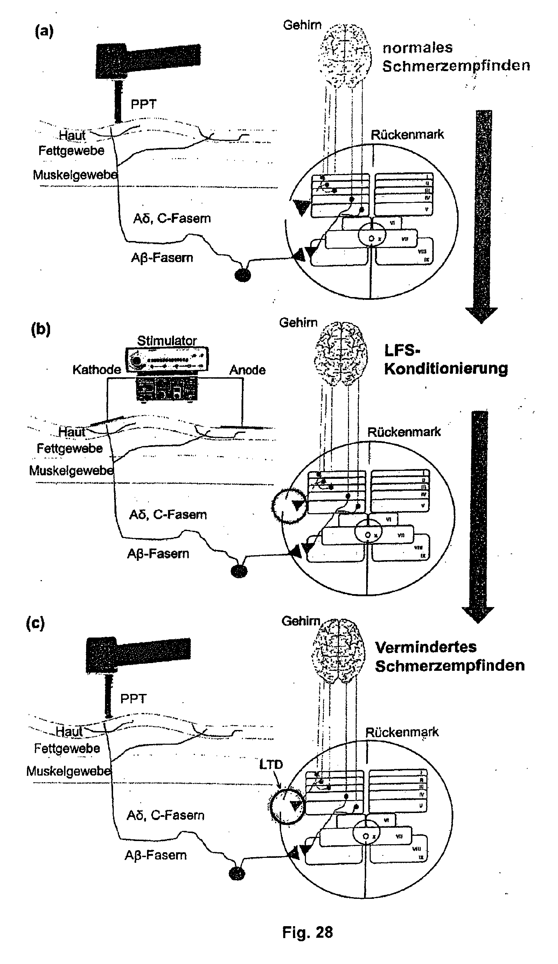

Fig. 28 zeigt eine synaptische Langzeitdepression (LTD) nach niederfrequenter Stimulation. In Fig. 28(a) ist eine normale Schmerzwahrnehmung gezeigt. Nach Reizapplikation (hier Druck) wird Aδ und C-Faser vermittelt das Signal auf Rückenmarksebene (Lamina I,II,III) vom primären auf das sekundäre Neuron umgeschaltet. Das Signal wird im Gehirn als normale Schmerzempfindung (hier Druckschmerz=PPT) wahrgenommen. In Fig. 28(b) ist die Situation nach einer elektrischen niederfrequenten kutanen Stimulation dargestellt. Es kommt an der Umschaltstelle zwischen erstem und zweitem Neuron zur Konditionierung mit anschließender LTD. In Fig. 28(c) ist die Reizapplikation nach vorheriger Konditionierung dargestellt. Es kommt zu einer verminderten Schmerzempfindlichkeit.

Die in der hier dargestellten Studie festgestellte thermische Hypästhesie und mechanische Hypoalgesie nach LFS lässt vermuten, dass C-Fasern auf spinale Neurone konvergieren, die für das Regelsystem für thermische und mechanische Wahrnehmung sowie thermischen und mechanischen Schmerz zuständig sind, oder dass diese zentralen Neuronengruppen von der gleichen Gruppe inhibitorischer Interneurone moduliert werden. Einerseits kann dieses System zu einer Reduzierung des zentralen Schmerzes, andererseits aber auch zu einer Verstärkung des Schmerzes und der Berührungssensitivität und somit zu einer Hyperalgesie führen. Dieses Phänomen konnte auch nach Capsaicininjektion beobachtet werden. Offensichtlich scheint bei diesem Mechanismus die Prozessierung verschiedener sensorischer Submodalitäten gemeinsam beeinflusst zu werden, da eine Hypästhesie entweder zusammen mit einer Hypoalgesie (wie hier nach 4Hz Konditionierung gezeigt) oder einer Hyperalgesie auftritt. Vermutlich steuern verschiedene Interneurone (gabaerge, glycinerge, opioiderge) präsynaptische oder postsynaptische Funktionen und sind dabei abhängig von Frequenz und Stärke des Stimulus.

In verschiedenen Surrogatmodellen wurde bereits gezeigt, dass schmerzhafte LFS zu einer LTD mit Zeichen einer Hypästhesie führt. Auch wurde beschrieben, dass durch hochfrequente Stimulation (HFS = high frequency stimulation) eine LTD ausgelöst werden kann. Dies würde allerdings heterosynaptische Prozesse voraussetzen, bei denen Interneurone ihre Entladungsfrequenz reduzieren. Eine weitere Überlegung wäre, dass es durch LFS zu einer LTP von hemmenden Interneuronen kommt. Dieser mögliche Mechanismus wurde bisher noch nicht experimentell untersucht.

Auch zentrale Mechanismen können zur Auslösung von LTD beitragen. So konnte beispielsweise gezeigt werden, dass durch schmerzhafte Stimulation die Verarbeitung taktiler afferenter Stimuli im Bereich 3b der kortikalen S1-Region zentral moduliert werden kann. Weiterführende Untersuchungen mit funktioneller Magnetresonanztomographie konnten nachweisen, dass es während niederfrequenter Stimulation zu zentralen Deaktivierungen in den Regionen S1 und S2 kommt. Vermutlich ist eine Kombination aus rostralen und spinalen Mechanismen für die Ausprägung von LTD verantwortlich.

### Vergleich Matrixelektrode (Elektrodenfeld und beabstandete Elektrode) vs. konzentrische Elektrode

Matrix- und konzentrische Elektrode weisen nach 4Hz Konditionierung für mechanische und thermische Schwellenwerte unterschiedliche Effekte auf. So zeigt die Reizung mit konzentrischer Elektrode für thermische Reize höhere Schwellenwerte, während die Reizung mit Matrixelektrode bei mechanischen Reizen ein größeres sensorisches Defizit auslöst.

Das Elektrodendesign scheint dabei ausschlaggebend zu sein. Durch die verschiedenartige Anordnung von Anode und Kathode und der dadurch entstehenden unterschiedlichen Stromdichten unter der jeweiligen Reizelektrode scheinen nozizeptive Schmerzfasern differenziert beeinflusst zu werden.

Für die konzentrische Elektrode konnte bereits in früheren Versuchen eine Änderung des schmerzverarbeitenden Systems im Vergleich zu konventionellen Elektroden gezeigt werden. Eigens durchgeführte Berechnungen nach der Methode der Finiten Elemente (FEM) der Felddichte der verwendeten Elektroden zeigen, dass die Matrixelektrode lokal höhere intrakutane Stromdichten aufweist als z.B. eine Gelelektrode. Somit ist das Auslösen von Aktionspotentialen im Bereich nozizeptiver Faserendigungen wahrscheinlicher als bei einer Gelelektrode. Die Stromdichte nimmt jedoch mit höherer Eindringtiefe stark ab. Vermutlich sind außerdem die punktuelle Kontaktfläche der Matrixelektrode und der größere Anodenabstand vorteilhafter, um gezielt mechanische Schwellenwerte zu beeinflussen.

Durch die Elektrodenplatzierung kommt es zusätzlich zu einer Beeinflussung des Stromflusses, was entsprechende Auswirkungen auf die Aktivierung von Hautafferenzen hat. Bei der konzentrischen Elektrode beträgt der Anoden-Kathodenabstand nur wenige Millimeter, möglicherweise ist dies ausschlaggebend um thermische Schwellenwertverschiebungen besser beeinflussen zu können. Beide Elektrodenarten beeinflussen Reize, die über Aδ- und C-Fasern vermittelt werden, die in den oberflächlichen Hautarealen zu finden sind. In dieser Studie konnte darüber hinaus für beide Elektrodentypen gezeigt werden, dass auch Aß-Faser-vermittelte Reize beeinflusst werden. Die Effektstärke für die resultierende taktile Hypästhesie war am größten - verglichen mit den Effekten auf die anderen QST-Parameter.

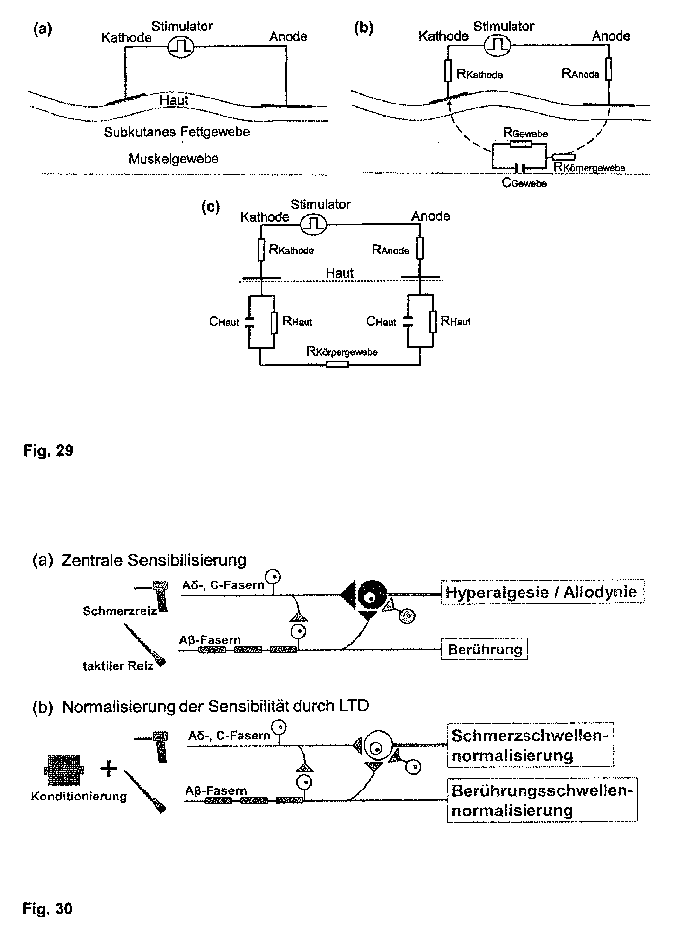

Niedrige Frequenzen eigenen sich besonders zur Auslösung von LTD. Bei diesen niedrigen Frequenzen und verwendeten monopolaren Pulsen kann der kapazitive Anteil der Haut vernachlässigt werden (Fig. 29). In der FE-Simulation der Elektrodeneffekte kann daher ein rein Ohm'scher Hautwiderstand angenommen werden.

Fig. 29 zeigt ein elektrisches Modell der Haut. Fig. 29(a) zeigt eine schematische Darstellung des Hautmodells; und Fig. 29(b) zeigt ein Ersatzschaltbild für einen Strompfad; sowie Fig. 29(c) eine technische Darstellung des Hautmodells zeigt.

Vermutlich kommt auch der Kathodenfläche bei der Auslösung von LTD eine entscheidende Rolle zu. So ist es wahrscheinlich, dass eine möglichst große Fläche eines nozizeltiven und/oder rezeptiven Feldes stimuliert werden muss, um LTD optimal zu induzieren, da auch eine Mindestzahl von Afferenzen gereizt werden muss, um diesen Mechanismus erst auszulösen. Grundsätzlich kann die Größe der Kontaktfläche einer Matrixelektrode leichter erweitert werden, was einen bautechnischen Vorteil gegenüber einer konzentrischen Elektrode darstellt.

Die meisten Patienten mit chronischen Schmerzsyndromen berichten über tief somatische oder viszerale Schmerzen (z.B. Gonarthrose, Tumorschmerz). Ausnahmen sind Erkrankungen mit Schmerzen, deren Ursprung im Hautbereich selbst liegt, wie dies zum Beispiel im Rahmen einer postherpetischen Neuralgie möglich ist. Die Ergebnisse der hier dargestellten Studie zeigen, dass eine Matrixelektrode besser für die Modulation von Tiefenschmerz geeignet ist. Da es bei einigen Schmerzerkrankungen zur Erhöhung der Schmerzsensibilität kommt, könnte eine Modulation von mechanischen Schmerzschwellen als Therapieunterstützung eingesetzt werden (Fig. 29). Ausgehend von dieser Einschätzung wird eine Matrixelektrode wahrscheinlicher als eine konzentrische Elektrode eine klinische Relevanz, auch beim Einsatz in der Therapie chronischer Schmerzsyndrome, besitzen. Fig. 30 zeigt eine schematische Darstellung mit: (a) zentrale Sensibilisierung mit Symptomen der Hyperalgesie und Allodynie bei Schmerzpatienten und (b) möglichem Mechanismus zur Normalisierung der Sensibilität durch LTD nach Konditionierung mit einer Matrixelektrode.

Im Gegensatz hierzu ist die Beeinflussung thermischer Wahrnehmungs- und Schmerzschwellen wahrscheinlich nur von geringer klinischer Bedeutung, beispielsweise in der Tumortherapie bei Patienten mit Kältehyperalgesie nach Cisplatin-Therapie. Weitere Untersuchungen mit abgewandelten Elektrodenmodellen mit optimierten Konfigurationen könnten in Zukunft Aussagen über deren Effektivität und über die räumliche Verteilung von Aktivierungsmustern zulassen und möglicherweise die pharmakologische Basistherapie von chronischen Schmerzerkrankungen unterstützen.

### Vergleich der Stimulationsfrequenzen 4Hz vs. 30Hz

In der Literatur wird zwischen einer nieder- und hochfrequenten Stimulation unterschieden. Als niederfrequente Stimulation werden Reizfrequenzen mit 0,5Hz bis 10Hz angegeben, während Frequenzen von 50 bis 100Hz betreffend TENS bereits zu den hochfrequenten Mustern zählen. Voraussetzung für die optimale Durchführung einer Untersuchung der Effekte einer niederfrequenten Stimulation (hier 4Hz) wäre somit als Kontrollfrequenz eine intermediäre Frequenz, die keinerlei relevante Effekte auf die untersuchten QST-Parameter zeigt (Sham-Frequenz). Auf der Basis eigener, hier nicht dargestellter Pilotdaten bei Messung der Druckschmerzschwelle (PPT), wurde von der Annahme ausgegangen, dass sich eine Stimulation mit 30Hz als optimale Sham-Frequenz (Kontrollbedingung ohne relevante Effekte) eignet. Dies konnte nach Auswertung der doppel-blind und als Cross-over Studie durchgeführten Untersuchung im Rahmen dieser Studie nur bedingt bestätigt werden.

Eine 30Hz-Stimulation mit der Matrixelektrode zeigte für die Druckschmerzschwelle (PPT) und thermischen Wahrnehmungsschwellen Werte, die sehr gut mit der Baseline-Bedingung vergleichbar waren. Für zukünftige Untersuchungen im humanen Surrogatmodell sollten daher für thermische Schwellenwertbestimmungen und für die Bestimmung der PPT eine Matrixelektrode mit 30Hz als Shamfrequenz verwendet werden. Allerdings war diese Frequenz für andere sensorische Modalitäten keine optimale Sham-Frequenz und führte ebenfalls zu deutlichen Schwellenerhöhungen der mechanischen Wahrnehmungsschwellen (MDT, Berührung und VDT, Vibration) mehr als der mechanischen Schmerzschwellen für Nadelreize. Diese LTD-Effekte waren nach 4Hz-Stimulation in der hier dargestellten Studie jedoch deutlicher als bei der 30Hz-Reizung ausgeprägt, was die Vermutung nahe legt, dass für die zuletzt genannten Parameter eine noch höhere Reizfrequenz als 30Hz eine optimale Shamstimulation darstellen könnte. Dieser Befund ist in Einklang mit anderen Experimenten, die zeigen, dass niederfrequente Stimulation zu synaptischer Langzeitdepression (LTD), aber hochfrequente Stimulation zu synaptischer Langzeitpotenzierung führt. Dieser Effekt mag jedoch für die verschiedenen sensorischen Kanäle jeweils unterschiedliche Frequenzen als Shamfrequenzen voraussetzen, die jeweils verschieden, aber immer zwischen dem Bereich der niedrigen und der hohen Frequenzen angesiedelt sind.

### Faktorenanalyse des somatosensorischen Phänotyps nach 4Hz-Konditionierung mit Matrixelektrode

Nachdem in der hier dargestellten Studie die deutlichsten Effekte, einschließlich einer Wirkung auf die Tiefenschmerzsensitivität, nur nach 4Hz-Stimulation mit der Matrixelektrode erzielt werden konnten, erfolgte eine Subanalyse in Form einer Faktorenanalyse nur für dieses Reizparadigma. Die Faktorenanalyse beschreibt dabei die Veränderungen aller untersuchten QST-Parameter nach konditionierender 4Hz-Stimulation gegenüber der Baseline-Bedingung. QST-Parameter, die nach Konditionierung ähnliche Effekte zeigten, gruppieren entsprechend auf einem Faktor im Rahmen einer multivariaten Faktorenanalyse (Varimax-Methode). Die beste Aufklärung der Gesamtvarianz gelang in einem 4-faktoriellen Modell. Dieser Befund überrascht nicht, da sich die QST-Parameter grundsätzlich an der Untersuchung von vier verschiedenen Bereichen der Somatosensorik ausrichten. QST untersucht hier allgemein die vier Bereiche thermischer und mechanischer Wahrnehmungs- und Schmerzschwellen. Passend hierzu gruppierten sich die QST-Parameter weitgehend konsistent. Auffallend waren zwei Befunde, die hier diskutiert werden sollen.

Zunächst soll auf die Abnahme der Tiefenschmerzempfindlichkeit nach 4Hz-Reizung mit einer Matrixelektrode hingewiesen werden. Anders als für alle anderen QST-Parameter kann dieser Effekt nicht allein als ein homotoper spinaler Effekt erklärt werden, also nicht als Effekt, der auf den Reizort beschränkt bleibt. Die veränderte Tiefenschmerzempfindlichkeit nach kutaner Stimulation kann im hier dargestellten Experiment nur auf einer Ebene erklärt werden, wo beide peripheren Systeme zusammenlaufen und die Effekte auf das eine System zu einer Performance-Änderung des anderen führen. Die Veränderung der Tiefenschmerzempfindlichkeit stellt hier einen neuartigen Befund dar, der am ehesten über heterosynaptische Plastizität an der Übertragungsstelle zwischen der peripheren Nervenendigung im Hinterhorn des Rückenmarks und spinalen WDR-Neuronen (WDR = wide dynamic range-Neurone) erklärt werden kann, die zusätzlich unter modulatorischer Kontrolle von inhibierenden Interneuronen sowie aus dem Hirnstamm absteigenden fazilitierenden und inhibierenden Projektionssystemen stehen.

Im Rahmen der Faktorenanalyse wäre hier zu erwarten gewesen, dass der Parameter "Druckschmerzsensitivität" PPT ein anderes Ladungsmuster auf die vier beschriebenen Faktoren zeigt oder gar einen eigenständigen fünften Faktor bildet. Dies war jedoch nicht der Fall. Wie die Fig. 27 zeigt, gruppiert PPT dennoch ganz nah bei den anderen mechanischen Schmerzschwellen für oberflächliche spitze Reize (MPT, MPS und WUR). PPT zeigt dabei ein sehr ähnliches Ladungsverhalten wie MPT. Beide Parameter repräsentieren mechanische Schmerzschwellen - ausgedrückt in auf die Haut und tiefere Gewebe aufgebrachte Kräfte, die zu einer ersten schmerzhaften Wahrnehmung führen. Die Ähnlichkeit im Ladungsverhalten dieser beiden Parameter deutet an, dass möglicherweise auch für andere QST-Parameter einer veränderten Oberflächensensibilität nicht ausschließlich homotope Effekte angenommen werden sollten. Möglicherweise können in die Veränderung der Schmerzempfindlichkeit gegenüber auch anderen QST-Parametern heterotope Effekte eingehen, was bei der Vielzahl der möglichen Interaktionen an der Schaltstelle eines WDR-Neurons auch wahrscheinlich ist.

Als zweite Besonderheit zeigte die Faktorenanalyse, dass die QST-Parameter MDT und VDT (Berührungsschwelle und Vibrationsschwelle) ein unterschiedliches Ladungsverhalten zeigten, obwohl beide Reize über Aß-Fasern vermittelt und über lemniskale Bahnen im Rückenmark ins Gehirn projiziert werden. Möglicherweise handelt es sich hier doch um zwei verschiedene zentrale Projektionswege ins Gehirn oder eine unterschiedliche spinale Beeinflussbarkeit dieser Phänomene auf der Ebene der ersten sensiblen Hinterhornneurone im Rückenmark.

### Zusammenfassung

Das Design der Studie diente der Etablierung eines humanen Surrogatmodells mit Einsatz niederfrequenter kutaner Neuromodulation zur Reduzierung der Schmerzempfindlichkeit. Dabei wurden zwei verschiedene Elektrodentypen hinsichtlich der Effektivität für die Induktion einer synaptischen Langzeitdepression (LTD) nach niederfrequenter 4Hz Stimulation verglichen. Im Rahmen einer vollständig balancierten, doppel-blinden, randomisierten Cross-over-Studie wurden 16 gesunde Probanden vor und nach niederfrequenter Reizung mit 4Hz und 30Hz mit beiden Elektrodenarten untersucht. Vor und nach Konditionierung wurde jeweils ein vollständiges somatosensorische Profil mittels Durchführung einer Quantitativen Sensorischen Testung (QST) erhoben. Dabei wurden in 7 Tests zusammen 13 verschiedene QST-Parameter erfasst mit Bestimmung thermischer und mechanischer Wahrnehmungs- und Schmerzschwellen. Es fanden sich folgende Hauptbefunde:
1) Durch niederfrequente Stimulation konnte das somatosensorische Profil gesunder Probanden verändert werden. Es fanden sich thermische und mechanische Hypästhesien und Hypoalgesien, speziell eine Hypoalgesie für spitze und auch stumpfe mechanische Reize.
2) Nach Induktion von LTD ähnelten sich die sensorischen Veränderungen zwischen der Matrix- und konzentrischen Elektrode mit dem Unterschied, dass nur die Matrixstimulation auch die Tiefenschmerzsensitivität reduzierte.
3) Eine 4Hz-Konditionierung zeigte sich einer 30Hz-Konditionierung überlegen. 30Hz stellen für die Druckschmerzschwelle und thermischen Wahrnehmungsschwellen eine optimale Sham-Frequenz dar, was für die anderen QST-Parameter nicht zutraf.
4) Die hier dargestellte Studie konnte erstmals am Menschen neben lokalen homotopen auch heterotope LTD-Effekte mit einer auch reduzierten Druckschmerzempfindlichkeit tieferer Gewebe zeigen.

Zudem wurden im Rahmen einer nachfolgend dargestellten Kurzstudie Zwei-Punkt-Diskriminations-Tests durchgeführt.

Mit Hilfe des Zwei-Punkt-Diskriminations-Tests wurden die rezeptiven Felder am Unterarm, Knie und unteren Rücken für mechanische und elektrische Reize untersucht. Die mechanischen Reize wurden mit einem neurologischen Zirkel und die elektrischen Reize mit zwei einzelnen Textilelektrodenpins ausgelöst. Die Pins hatten als Elektrodenform die Form der Elektroden für das Elektrodenfeld. Die Elektrodenpins dienten als Kathoden. Es wurde zudem eine flächige Gegenelektrode als Anode verwendet.

Um die Länge und Breite der rezeptiven Felder auszumessen, wurden den Probanden auf den Unterarm, das Knie und den unteren Rücken Markierungen im Abstand von 1 cm gesetzt. Um den minimalen Abstand, bei dem zwei identische Reize noch getrennt voneinander wahrgenommen werden, zu bestimmen, wurden die beiden identischen Reize gleichzeitig mit identischem Druck auf die Haut aufgesetzt. Es wurde mit einem weiten Abstand begonnen. Anschließend wurde der Abstand der beiden Reize immer um 1 cm verkürzt, bis die Reize nicht mehr als zwei Einzelreize wahrgenommen wurden. Dieser Abstand wurde notiert. Dann wurde der Abstand immer um 1cm erweitert, bis die Reize wieder als zwei getrennte Reize wahrgenommen wurden. Auch dieser Wert wurde notiert. Dieses Vorgehen erfolgte solange, bis die Schwelle eindeutig bestimmt werden konnte. Wurden die Reize noch bei 1cm Abstand getrennt wahrgenommen, wurde der Abstand weitere zweimal um jeweils die Hälfte des vorangegangen Abstandes verkürzt. Die Position der flächigen Gegenelektrode wurde nicht verändert, wobei ein Abstand von mehreren cm gewählt wurde.

### Ergebnisse:

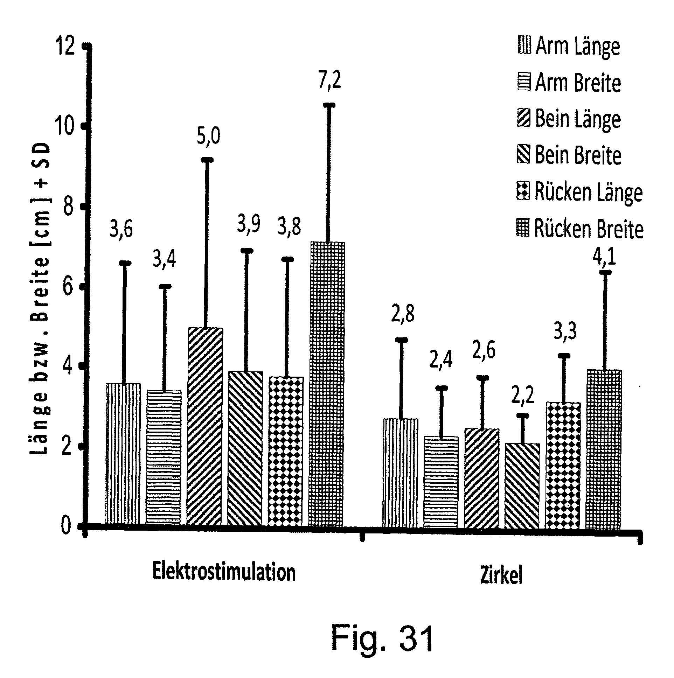

### Die Ergebnisse sind in Fig. 31 grafisch dargestellt.

Die Ergebnisse deuten darauf hin, dass die rezeptiven Felder für den mechanischen Reiz mit dem neurologischen Zirkel etwas kleiner sind als für die elektrische Stimulation. Auf dem Unterarm ist das rezeptive Feld des mechanischen Reizes etwa 1 cm kürzer und schmaler als für den elektrischen Reiz. Die Größe des rezeptiven Feldes für diesen Reiz ist in etwa identisch mit der Literatur. Die Ergebnisse für das Knie deuten darauf hin, dass das rezeptive Feld für den mechanischen Reiz nur etwa halb so groß ist, wie für den elektrischen Reiz. Am Rücken unterscheiden sich die rezeptiven Felder für diese beiden Reize hauptsächlich in der Breite. Für den elektrischen Reiz ist das Feld etwa 7,2cm und für den mechanischen Reizes etwa 4,1cm breit. In der Literatur wird für den elektrischen Reiz eine Breite von etwa 6cm angegeben. Insgesamt betrachtet sind die rezeptiven Felder für den elektrischen Reiz für den Unterarm und Knie ähnlich groß, während das rezeptive Feld am unteren Rücken fast doppelt so breit wie lang ist. Damit orientiert sich das Feld entlang des Dermatoms. Für den mechanischen Reiz ähneln sich ebenfalls die rezeptiven Felder am Unterarm und Knie. Das rezeptive Feld am unteren Rücken ist nur geringfügig größer.

Der maximale Abstand, den zwei separate Elektroden eines Elektrodenfelds für den Unterarm, das Knie oder den unteren Rücken haben dürfen, ohne die klinische Effizienz zu verringern, kann aus dem Zwei-Punkt-Diskriminationstest abgeleitet werden. Die im Zwei-Punkt-Diskriminationstest ermittelte Schwelle gibt die Größe des rezeptiven Feldes an. Der Abstand von Elektroden des Elektrodenfelds darf für die jeweilige Region maximal die Hälfte des rezeptiven Feldes betragen, um sicherzustellen, dass ausreichend Elektroden des Elektrodenfelds über einem rezeptiven Feld liegen, um die darunterliegenden Nervenenden zuverlässig anzusprechen.

Die Ergebnisse deuten darauf hin, dass die rezeptiven Felder für den mechanischen Reiz mit dem neurologischen Zirkel etwas kleiner sind als für die elektrische Stimulation. Da der neurologische Zirkel und die elektrische Stimulation dieselben Nervenfasern aktivieren, sollte der Abstand nicht größer als die Hälfte der Größe des rezeptiven Feldes des neurologischen Zirkels sein. Der Test zeigte, dass das rezeptive Feld am Knie in seiner Größe dem rezeptiven Feld am Unterarm gleicht. Für diese beiden Areale können folglich Elektroden verwendet werden, die in ihren Abständen identisch sind. Für den unteren Rücken zeigte die Untersuchung, dass die Abstände der Elektroden in der Breite größer sein dürfen als in der Länge.

Es wurde zudem eine offen, randomisierte und kontrollierte Gonathrose-Studie durchgeführt, mit der statistische Belege für die Wirksamkeit der erfindungsgemäßen Elektrodenstimulation gezeigt werden konnten. Es gab signifikante Ergebnisse für die Reduktion bei Ruhe-, Bewegungs- und Nachtschmerz. Die Ergebnisse der erfindungsgemäßen Vorrichtung mit elektrischer Stimulation wurden mit den Ergebnissen einer nur Druck ausübenden Bandage verglichen und es zeigte sich, dass die erfindungsgemäße elektrische Stimulation beim Ruhe- und Nachtschmerz wirkungsvoller war als gegenüber der nur Druck ausübenden Bandage.

## Patentansprüche

1. Elektrodenanordnung mit einem Elektrodenfeld sowie mindestens einer zu dem Elektrodenfeld beabstandeten, weiteren Elektrode zur Behandlung von Schmerzen eines Menschen oder eines Tieres, wobei die Elektrodenanordnung mindestens mit einem Teil zur Anlage an dem Menschen oder an das Tier ausgebildet ist, und eine Vibrationseinrichtung umfasst.

2. Elektrodenanordnung mit einem Elektrodenfeld sowie mindestens einer zu dem Elektrodenfeld beabstandeten, weiteren Elektrode zur Behandlung von Schmerzen eines Menschen oder eines Tieres, wobei die Elektrodenanordnung mindestens mit einem Teil zur Anlage an dem Menschen oder an das Tier ausgebildet ist, und im Bereich des Elektrodenfeldes ein Druckkörper vorgesehen ist.

3. Elektrodenanordnung nach Anspruch 1 oder 2, wobei die Elektroden des Elektrodenfeldes die Kathoden bilden und die weitere Elektrode die Anode bildet.

4. Elektrodenanordnung nach einem der Ansprüche 1 bis 3, wobei der Abstand zwischen Elektrodenfeld und weiterer Elektrode weniger als 10cm beträgt.

5. Elektrodenanordnung nach einem der Ansprüche 1 bis 4, wobei die Elektroden des Elektrodenfelds eine halbkugelförmige Oberfläche zur Anlage auf die Haut aufweisen.

6. Elektrodenanordnung nach einem der Ansprüche 1 bis 5, wobei die weitere Elektrode flächig ausgestaltet ist.

7. Elektrodenanordnung nach einem der Ansprüche 1 bis 6, wobei die Elektroden des Elektrodenfelds auf einer Trägerstruktur ausgebildet sind.

8. Elektrodenanordnung nach Anspruch 7, wobei die Trägerstruktur (31) ein elektrisch nichtleitendes Material umfasst, mittels dem die Elektroden voneinander isoliert sind.

9. Elektrodenanordnung nach Anspruch 7 und 8, wobei die Trägerstruktur (31) ein der Haut zugewandtes Material umfasst, das saugfähig ist.

10. Elektrodenanordnung nach einem der Ansprüche 1 bis 9, **dadurch gekennzeichnet, dass** eine Steuereinheit (19, 35) vorgesehen ist, mittles der das Elektrodenfeld und/oder die weitere Elektrode mit elektrischen Impulsen beaufschlagbar ist, und bevorzugt auch für die Ansteuerung einer Vibrationseinrichtung ausgestaltet ist.

11. Elektrodenanordnung nach Anspruch 13 oder 14, wobei die Elektroden des Elektrodenfelds mit einem durch die Steuereinheit (19, 35) regelbaren Strom von 0,1 bis 30mA impulsförmig beaufschlagbar sind.

12. Elektrodenanordnung nach einem der Ansprüche 13 bis 15, wobei die Pulsdauer 100µs bis 500µs beträgt.

13. Elektrodenanordnung nach einem der Ansprüche 13 bis 16, wobei die Pulsfolgefrequenz 0,1 Hz bis 20Hz beträgt.

14. Elektrodenanordnung nach einem der Ansprüche 13 bis 17, wobei die Spannungsversorgung (34) der Steuereinheit (19, 35) über einen Akkumulator oder eine Batterie möglich ist, die eine maximale Spannung im zweistelligen Volt-Bereich benötigt.

15. Vorrichtung zur Behandlung von Schmerzen eines Menschen oder eines Tieres mit einer Elektrodenanordnung, die ein Elektrodenfeld und mindestens eine hierzu beabstandete, weitere Elektrode sowie einen Druckkörper aufweist, wobei der Druckkörper so ausgestaltet ist, dass mindestens Teile der Elektrodenanordnung an den Körper des Menschen oder des Tieres gedrängt werden können.
